# EUROPEAN PATENT SPECIFICATION

(11) **EP 2 322 204 B1**
(45) Date of publication and mention of the grant of the patent: **25.10.2017**
(21) Application number: 09176204.7
(22) Date of filing: 17.11.2009
(51) Int. Cl.: A61K 38/17, A61P 35/00, A61P 31/18

(54) **Pharmaceutical compositions comprising an LRP1 receptor agonist for the treatment of cancer and HIV**
Pharmazeutische Zusammensetzungen mit einem LRP1-Rezeptoragonisten zur Behandlung von Krebs und HIV
Compositions pharmaceutiques comprenant un agoniste du récepteur LRP1 pour le traitement du cancer et du VIH

(43) Date of publication of application: 18.05.2011
(73) Proprietor: Fraunhofer-Gesellschaft zur Förderung der angewandten Forschung e.V., 80686 München (DE); Universität Leipzig, 04109 Leipzig (DE)
(72) Inventor: Birkenmeier, Gerd, 04103 Leipzig (DE); Schäfer, Angelika, 04205 Leipzig (DE); Hemdan, Nasr, 04103 Leipzig (DE); Buchold, Martin, 04317 Leipzig (DE); Lindner, Inge, 01069 Dresden (DE); Bigl, Marina, 04821 Polenz (DE); Gerdes, Wilhelm, 04275 Leipzig (DE)
(74) Representative: Bühler, Dirk

(56) References cited:
- WO-A1-01/57266
- WO-A2-2004/033657
- SAREDDY GANGADHARA REDDY ET AL: "Activation of Wnt/beta-catenin/Tcf signaling pathway in human astrocytomas." NEUROCHEMISTRY INTERNATIONAL SEP 2009, vol. 55, no. 5, September 2009 (2009-09), pages 307-317, XP002575833 ISSN: 1872-9754
- BAUM LARRY ET AL: "Low density lipoprotein receptor related protein gene amplification and 766T polymorphism in astrocytomas" NEUROSCIENCE LETTERS, vol. 256, no. 1, 30 October 1998 (1998-10-30), pages 5-8, XP002575834 ISSN: 0304-3940

### SEQUENCE LISTING

<110> Fraunhofer-Gesellschaft zur Förderung der angewandten Forschung e.V.
<120> Pharmaceutical compositions comprising an LRP1 receptor agonist for the treatment of cancer and HIV
<130> F8755/DB
<160> 24
<170> PatentIn version 3.3
<210> 1
   <211> 19
   <212> DNA
   <213> Artificial
<220>
   <223> oligonucleotide primer
<400> 1
   agccacatcg ctcagacac 19
<210> 2
   <211> 19
   <212> DNA
   <213> Artificial
<220>
   <223> oligonucleotide primer
<400> 2
   gcccaatacg accaaatcc 19
<210> 3
   <211> 20
   <212> DNA
   <213> Artificial
<220>
   <223> oligonucleotide primer
<400> 3
   gatgagacac acgccaactg 20
<210> 4
   <211> 18
   <212> DNA
   <213> Artificial
<220>
   <223> oligonucleotide primer
<400> 4
   cggcactgga actcatca 18
<210> 5
   <211> 19
   <212> DNA
   <213> Artificial
<220>
   <223> oligonucleotide primer
<400> 5
   gaacatcaag cgagccaag 19
<210> 6
   <211> 21
   <212> DNA
   <213> Artificial
<220>
   <223> oligonucleotide primer
<400> 6
   tggctcagag aggtcaaaac a 21
<210> 7
   <211> 20
   <212> DNA
   <213> Artificial
<220>
   <223> oligonucleotide primer
<400> 7
   atccgaaagg cacaagaaga 20
<210> 8
   <211> 21
   <212> DNA
   <213> Artificial
<220>
   <223> oligonucleotide primer
<400> 8
   gactcggaac tgagctcaca a 21
<210> 9
   <211> 21
   <212> DNA
   <213> Artificial
<220>
   <223> oligonucleotide primer
<400> 9
   tcatgggcta tgaagatgag g 21
<210> 10
   <211> 22
   <212> DNA
   <213> Artificial
<220>
   <223> oligonucleotide primer
<400> 10
   tcatatccca gcgcttaact tt 22
<210> 11
   <211> 18
   <212> DNA
   <213> Artificial
<220>
   <223> oligonucleotide primer
<400> 11
   cgctggaact gtcccact 18
<210> 12
   <211> 18
   <212> DNA
   <213> Artificial
<220>
   <223> oligonucleotide primer
<400> 12
   aacgccgttt ctcgacag 18
<210> 13
   <211> 18
   <212> DNA
   <213> Artificial
<220>
   <223> oligonucleotide primer
<400> 13
   aactgcacca ccgtccac 18
<210> 14
   <211> 19
   <212> DNA
   <213> Artificial
<220>
   <223> oligonucleotide primer
<400> 14
   ccgactccct ggtagcttt 19
<210> 15
   <211> 20
   <212> DNA
   <213> Artificial
<220>
   <223> oligonucleotide primer
<400> 15
   taagcccagg agttgctttg 20
<210> 16
   <211> 25
   <212> DNA
   <213> Artificial
<220>
   <223> oligonucleotide primer
<400> 16
   ctgaacaggg ttattcatac ctagc 25
<210> 17
   <211> 19
   <212> DNA
   <213> Artificial
<220>
   <223> oligonucleotide primer
<400> 17
   gcgaatccac aacaacagg 19
<210> 18
   <211> 20
   <212> DNA
   <213> Artificial
<220>
   <223> oligonucleotide primer
<400> 18
   tccagcatgt cttgaactgg 20
<210> 19
   <211> 20
   <212> DNA
   <213> Artificial
<220>
   <223> oligonucleotide primer
<400> 19
   gctttcagtt gagctgacca 20
<210> 20
   <211> 22
   <212> DNA
   <213> Artificial
<220>
   <223> oligonucleotide primer
<400> 20
   aagtccaaga tcagcagtct ca 22
<210> 21
   <211> 20
   <212> DNA
   <213> Artificial
<220>
   <223> oligonucleotide primer
<400> 21
   cccgggacaa cgtttattac 20
<210> 22
   <211> 20
   <212> DNA
   <213> Artificial
<220>
   <223> oligonucleotide primer
<400> 22
   gctggctcaa gtcaaagtcc 20
<210> 23
   <211> 21
   <212> DNA
   <213> Artificial
<220>
   <223> oligonucleotide primer
<400> 23
   ggtggaggag aagaagacca g 21
<210> 24
   <211> 18
   <212> DNA
   <213> Artificial
<220>
   <223> oligonucleotide primer
<400> 24
   ggcatcaggc tccacagt 18

## Description

### FIELD OF THE INVENTION

The present invention provides a pharmaceutical composition comprising an LRP1 receptor agonist for use in the treatment of cancer as claimed in independent claim 1 and an in vitro method as claimed in independent claim 6. The present invention also provides a pharmaceutical composition comprising an LRP1 receptor agonist for use in the treatment of an HIV-infection as claimed in independent claim 2. Furthermore, the present invention relates to the therapeutic use of LRP1 receptor agonists as claimed in independent claim 4 and a kit of parts as claimed in independent claim 9 comprising the pharmaceutical composition according to the invention.

### BACKGROUND OF THE INVENTION

Alpha-2-macroglobulin, also known as α₂-macroglobulin (abbreviated A2M or α2M), is a highly abundant, high molecular mass plasma protein.

Nascent A2M is known to inactivate self or foreign proteases, e.g. proteases accompanying tumor cell invasion such as plasmin, urokinase-type plasminogen activator (uPA) and metalloproteases (MMP).

Binding to proteases in vivo induces a conformational change in the A2M molecule that results in trapping of the protease within a cage-like interior cavity and appearance of distinct binding sites for transforming growth factor (TGF)-β, cytokines and other growth regulating ligands. Furthermore, said transformed form of A2M, which is referred to as activated alpha-2-macroglobulin or A2M* (also α2M* or Fα2M), is capable of binding to the transmembrane receptor LRP1 (also known as CD91), a member of the low-density lipoprotein receptor superfamily. In vitro, A2M may also be activated by small amines, such as e.g. methylamine. Upon binding to LRP1, the A2M*-protease-growth factor complexes are endocytosed and delivered to endosomes and lysosomes for degradation, a process commonly referred to as "clearance of activated A2M".

Wnt proteins are secreted lipid-modified signaling proteins that control a variety of developmental processes including e.g. cell fate specification, proliferation, polarity and migration. Upon binding to cell surface receptors called Frizzeled (Fz), Wnt proteins trigger intracellular signaling cascades, which play a crucial role in embryonic development and in the regeneration of tissues in adult organism.

The binding of Wnt to Frizzeled can activate signaling via three sub-pathways: the canonical Wnt/ß-catenin pathway, Wnt/Ca2+ pathway or Wnt/planar cell polarity pathway.
The best studied pathway is the canonical Wnt/ß-catenin pathway. Said pathway describes a series of events that occur when Wnt proteins bind to Frizzled, causing the receptors to activate Dishevelled family proteins and ultimately resulting in a change in the amount of β-catenin that reaches the nucleus where it interacts with transcription factors and initiates Wnt target gene transcription.

Perturbations in the Wnt/β-catenin pathway, e.g. overactivation of said pathway, have been linked to a variety of human diseases, such as cancer. Several mechanisms of activation of the canonical Wnt pathway in tumors have been discovered; e.g. silencing of the genes that encode the inhibitory Wnt ligands SFRP and DKK or overexpression of Wnt proteins, Frizzled or Dishevelled. Furthermore, mutations that promote constitutive activation of the Wnt signaling pathway have been shown to lead to cancer.

Cancer is a class of diseases in which a group of cells display uncontrolled growth, invasion and sometimes metastasis. These three malignant properties of cancers differentiate them from benign tumors, which are self-limited and do not invade or metastasize.
Cancer is a major health problem causing about 13% of all deaths worldwide. According to the American Cancer Society, 7.6 million people died from cancer in the world during 2007. Deaths from cancer are projected to continue rising, with an estimated 12 million deaths in 2030.

The development of novel therapies to combat cancer therefore remains an essential task.

Acquired immunodeficiency syndrome (AIDS), one of the most important infectious diseases in humans, poses another global health problem. AIDS, which is caused by human immunodeficiency virus (HIV), is a condition in which the immune system begins to fail, leading to life-threatening opportunistic infections.

HIV infection in humans is considered pandemic by the World Health Organization. From 1981 to 2006, AIDS killed more than 25 million people.

Current treatments of HIV-infection and AIDS mainly include highly active antiretroviral therapy (HAART), which reduces the viral load in HIV-infected patients. Antiretroviral drugs include e.g., nucleoside and nucleotide reverse transcriptase inhibitors, non-nucleoside reverse transcriptase inhibitors and HIV protease inhibitors.

However, many patients treated by antiretroviral therapy develop multiple metabolic complications and are at risk for developing chronic diseases.

There is thus an ongoing need for novel therapies against HIV.

A small percentage of HIV-infected individuals remain asymptomatic for a long period post infection, presumably due to a vigorous immune response which may contribute to long term non progression. The LRP1 receptor has been found to be significantly up-regulated on monocytes of HIV-infected non-progressors, indicating that high LRP1 expression plays an important role in protecting cells against HIV infection. HIV-1 antigen uptake and cross-presentation via LRP1-mediated endocytosis may e.g. contribute to mechanisms underlying a host anti-HIV-1 defense.

WO 01/57266 in the name of Hyseq, Inc discloses novel polynucleotides and polypeptides encoded by such polynucleotides and mutants or variants thereof that correspond to novel human secreted apha-2-macroglobulin-like polypeptides.

WO2004/033657 in the name of Antigenics, Inc and the University of Connecticut Health Center discloses compositions and methods for the use of natural and recombinant p95 forms and fragments as heat shock protein binding proteins. The application also discloses CD91 polypetide fragments that comprise at least p95 and additional contiguous sequence from domain II, III, and IV of CD91.

### OBJECTIVE AND SUMMARY OF THE INVENTION

It is one object of the present invention to provide a pharmaceutical composition suitable for use in the treatment of a cancer with an up-regulated or constitutively active Wnt signaling pathway.

It is another objective of the present invention to provide a method for identifying a compound suitable as a therapeutic agent for treating cancer with an up-regulated or constitutively active Wnt signaling pathway.

It is a further object of the present invention to provide a pharmaceutical composition suitable for use in the treatment of an HIV infection in a subject.

These and other objectives as they will become apparent from the ensuing description and claims are attained by the subject matter of the independent claims. Some of the preferred embodiments are defined by the dependent claims.

In a first aspect the present invention provides a pharmaceutical composition comprising an LRP1 receptor agonist for use in the treatment of cancer as claimed in independent claim 1.

In another aspect the invention relates to a pharmaceutical composition comprising an LRP1 receptor agonist for use in the treatment of an HIV-infection as claimed in independent claim 2.

In a further aspect the invention provides in independent claim 4 the use of an LRP1 receptor agonist or of a pharmaceutical composition according to the invention for the manufacture of a medicament for the treatment of the cancers as listed in independent claim 4 and an HIV infection

In yet another aspect the present invention relates to an in vitro method for identifying a therapeutic agent for treating cancer as claimed in independent claim 6.

In another aspect the invention relates to a kit of parts as claimed in independent claim 9 comprising the pharmaceutical composition according to the invention and diagnostic reagents suitable for determining whether the Wnt-signaling pathway is up-regulated or constitutively active in a cell.

### DESCRIPTION OF THE FIGURES

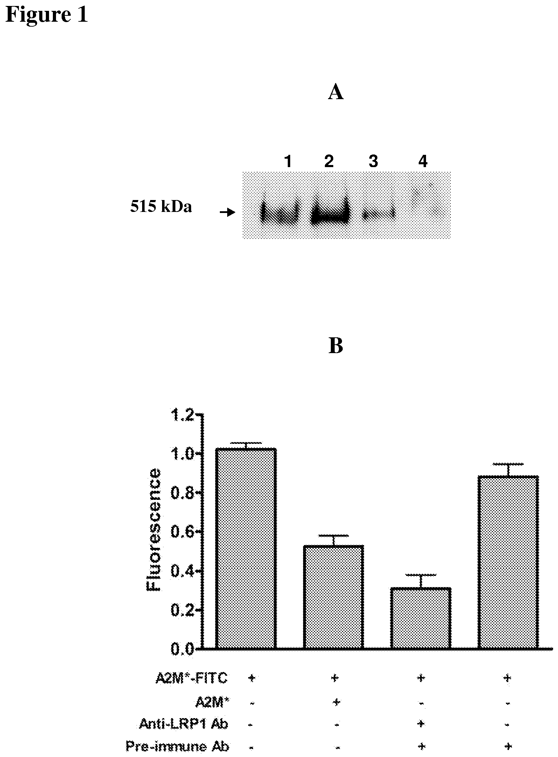

**Figure 1****.** Immunodetection of LRP1 protein in 1321N1 astrocytoma cells and analysis of cellular uptake of FITC-labeled A2M*. A) Cellular extracts of human astrocytoma (86030102; ECACC), fibroblasts, LNCaP (DSMZ No ACC 256) and DU-145 (DSM ACC 261) prostate cancer cells (50 µg each, lanes 1-4, respectively) were subjected to SDS-PAGE and western blotting. B) Uptake of A2M*-FITC by astrocytoma, and competition by A2M* (2 µM) and anti-LRP1 Ab (2 µM). Pre-immune Ab (2 µM) was used as a control. Samples were measured in triplicates (n = 6; the asterisks donate significance at p< 0.05).

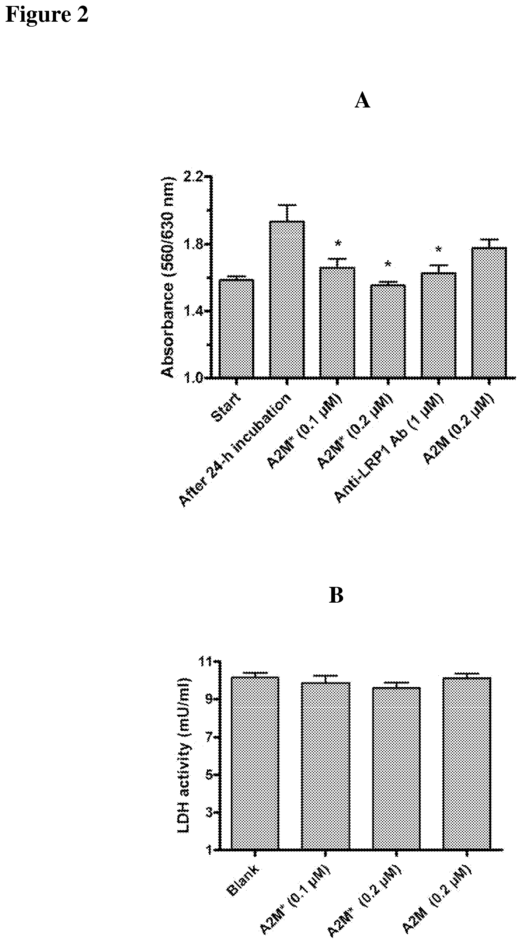

**Figure 2****.** A2M* reduces proliferation/vitality but does not affect LDH release of 1321N1 astrocytoma cells. A) Astrocytoma (5x10⁵ cells/ml: start) were cultured in complete medium for 24 h in the absence or presence of A2M*, A2M or anti-LRP1 Ab. Cell vitality was assessed by WST-1 assay. Data represent mean±S.D. of 6 independent experiments. B) LDH activity (mU/ml) was measured in the supernatant upon incubation of tumor cells with A2M* or native A2M. Columns represents means±S.D. of 3 replicates (n= 6; the asterisks donate significance at p < 0.05).

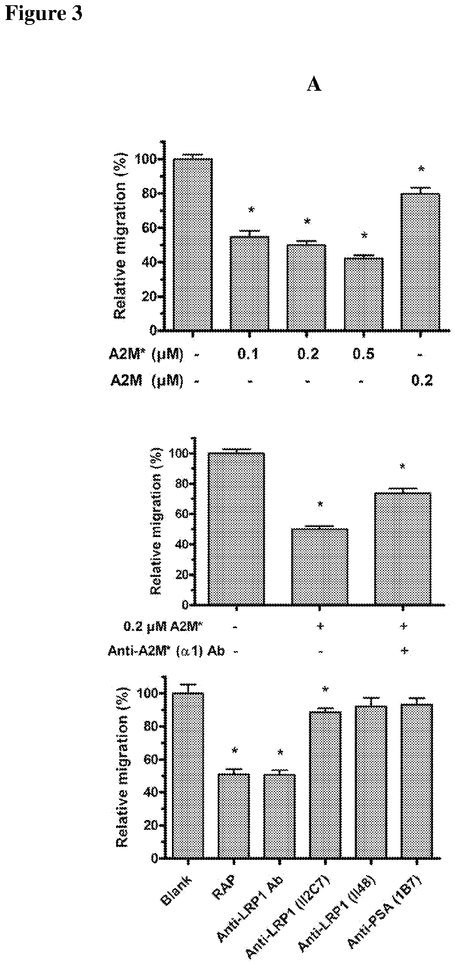

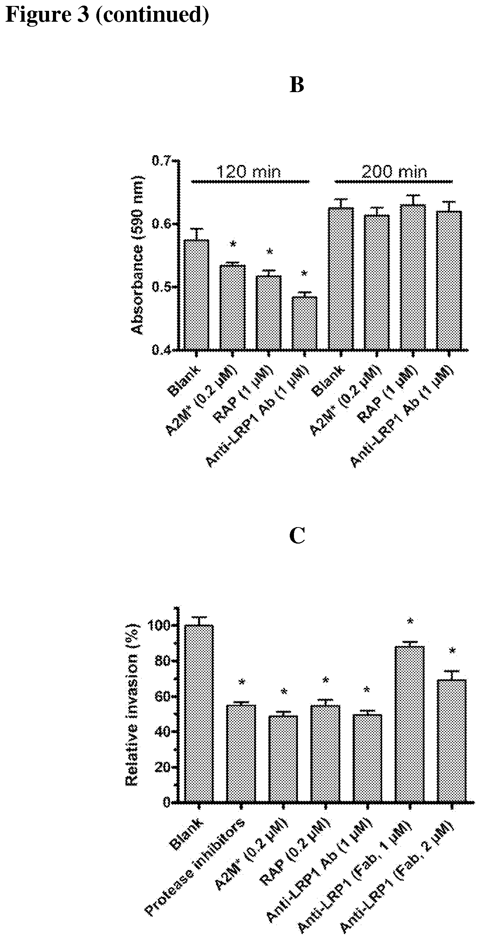

**Figure 3****.** A2M* and LRP1-ligands inhibits migration, adhesion and invasion of 1321N1 cells. A) Dependence of migration of tumor cells on the concentration of A2M and A2M* (upper panel), anti-A2M* mAb (clone α1-IIE7; 0.2 µM) directed against LRP1-binding domain (middle panel) and the receptor-associated protein (RAP) (0.2 µM), polyclonal anti-LRP1 Ab (1 µM) and various mAbs (0.2 µM) directed against the 515-kDa (II2C7) and 85-kDa (II4/8) receptor subunit in comparison to an irrelevant anti-PSA mAb (lower panel) in comparison to untreated cells (blank).
   B) The effect of A2M* and LRP1-ligands on adhesion of 1321N1 cells at gelantine-coated 96-well plates measured at 120 min and 200 min. Columns represent means±S.D. of triplicates (n= 6; the asterisks donate significance at p< 0.05). C) Invasion was analyzed by coating the porous inserts with 25 µl Matrigel prior to seeding. Cell migration was analyzed in the absence or presence of a protease inhibitor cocktail (5 µl/ml), A2M*, RAP, anti-LRP1 Ab and various concentrations of anti-LRP1 Ab-Fab fragments. Columns represent means±S.D. (n = 6, the asterisks donate significance at p< 0.05).

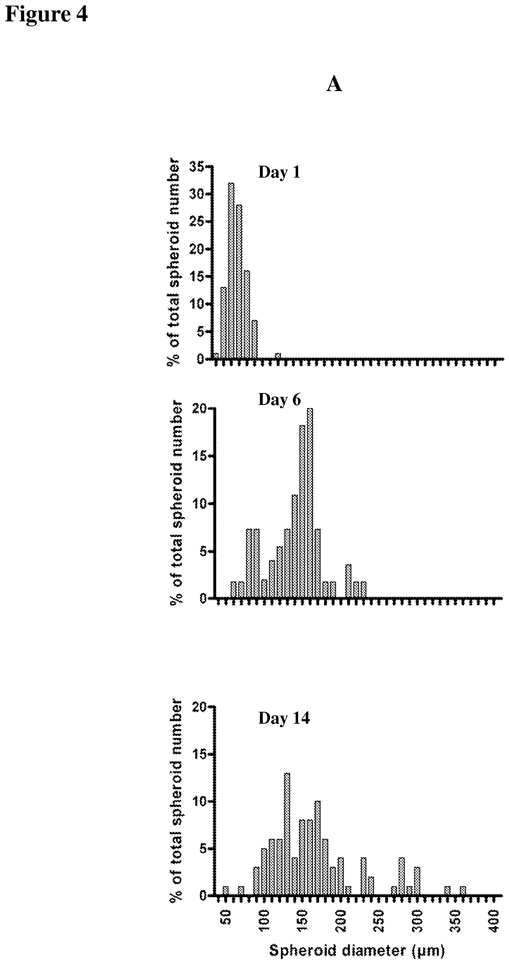

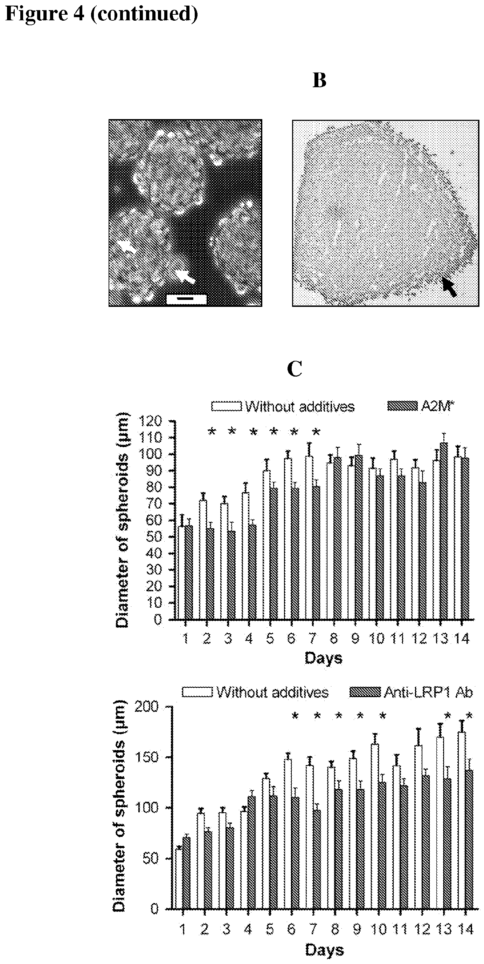

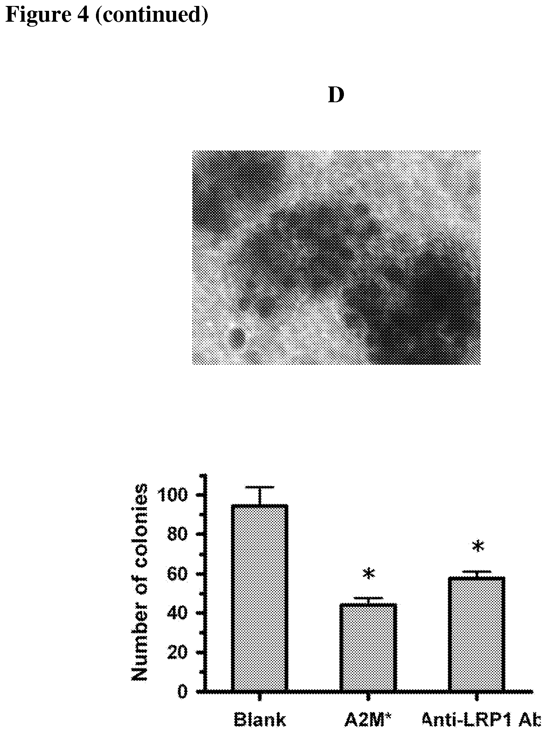

**Figure 4****.** Exposure to A2M* or anti-LRP1 Ab inhibits formation of tumor spheroids and cell colonies. A -C) Spheroid formation in the absence of A2M* and anti-LRP1: A) Size distribution throughout two weeks, B) Light microscopy of a growing spheroid; the arrow shows shedding of cells (bar= 5 µm) and immunostaining of LRP1 antigen in a 1-mm tumor spheroid; the arrow indicates the positive staining at the margin. C) Change in spheroids' size over time in the presence of 0.2 µM A2M* or 1 µM anti-LRP1 Ab. Plots represent the mean±S.D. (n = 70; the asterisks donate significance at p< 0.05). D) Representative view of cell colonies and inhibition of colony formation of 1321N1 cells in soft agar by A2M* and anti-LRP1 Ab. Columns represent means of colony counts±S.D. (n = 8; the asterisks donate significance at p < 0.05).

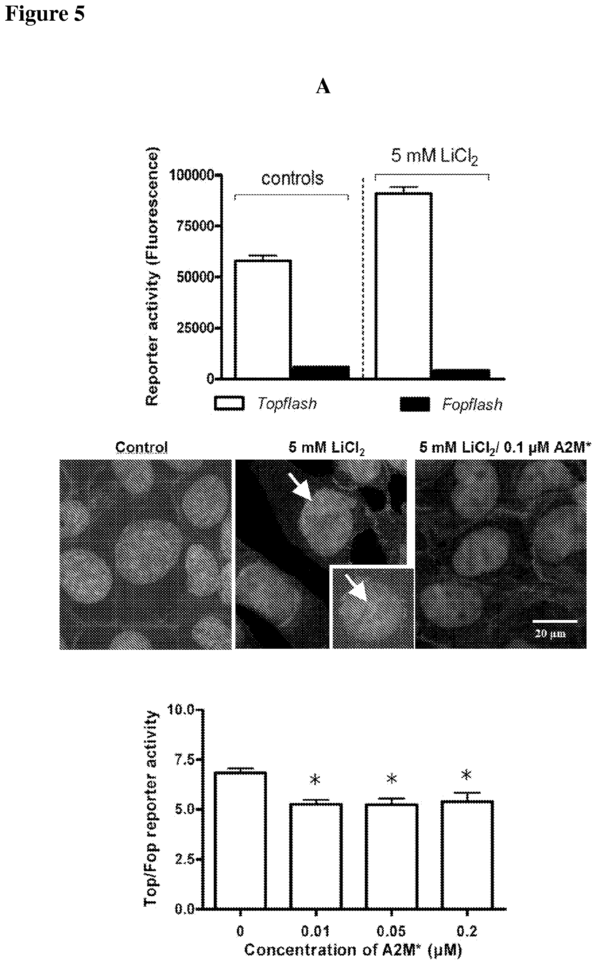

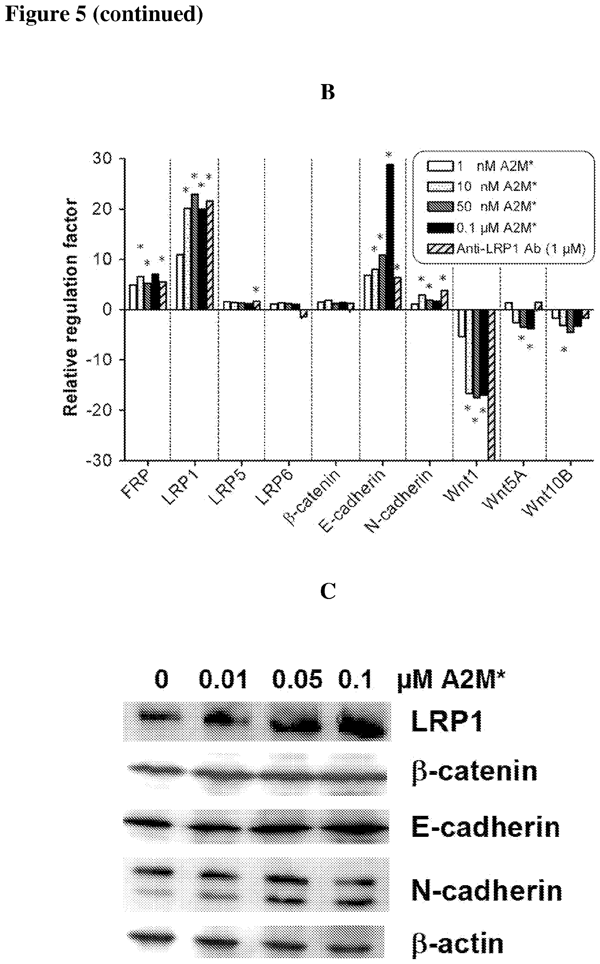

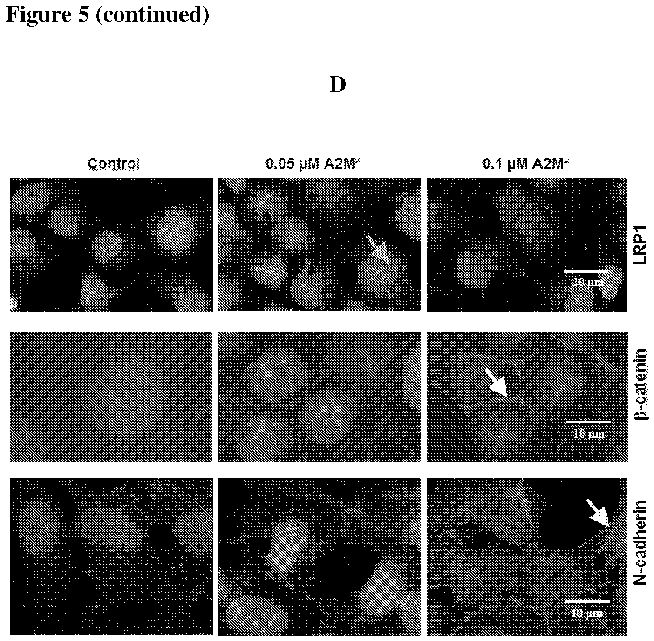

**Figure 5****.** Modulation of the Wnt/β-catenin signaling pathway by A2M* in 1321N1 astrocytoma cells. A) Activation of the constitutive Wnt/β-catenin pathway by LiCl₂ evaluated by Top*Flash*/Fop*flash*-reporter assay. The activity of Fop*flash*-reporter gene containing a mutated TCF-binding site was used as internal control (left). Immunochemical staining of β-catenin (white arrow) in untreated and cells treated with 5 mM LiCl₂ and with 5 mM LiCl₂/0.1 µM A2M*; the insert demonstrates the nuclear localization of β-catenin (middle). Attenuation of the reporter gene activity by A2M* (right). B) Relative expression of key genes of the Wnt/β-catenin signaling pathway following exposure to successive concentrations of A2M* or anti-LRP1 Ab (1 µM). Plotted is the up- or down-regulation factor relative to the untreated controls (GAPDH; assigned to 0). The asterisks indicate significance relative to controls. C) Western blot analysis of LRP1, β-catenin, E- and N-cadherin, and β-actin (control) in 1321N1 cells treated with A2M* (0-0.1µM). D) Immunostaining of LRP1 (arrow upper panel), β-catenin (the white arrow in the middle panel shows the relocated β-catenin in the plasma membrane) and N-cadherin (arrow lower panel) in astrocytoma cells treated with 0.05 µM and 0.1 µM A2M*.

### DETAILED DESCRIPTION OF THE INVENTION

Unless defined otherwise, all technical and scientific terms used herein have the same meanings as commonly understood by one of ordinary skill in the art.

The following definitions are introduced:
It is to be understood that the term "comprise", and variations such as "comprises" and "comprising" is not limiting. For the purpose of the present invention the term "consisting of" is considered to be a preferred embodiment of the term "comprising". If hereinafter a group is defined to comprise at least a certain number of embodiments, this is meant to also encompass a group which preferably consists of these embodiments only.

The terms "about" and "approximately" in the context of the present invention denote an interval of accuracy that a person skilled in the art will understand to still ensure the technical effect of the feature in question. The term typically encompasses a deviation from the indicated numerical value of ±10 % and preferably of ±5 %.

The determination of percent identity between two protein sequences is preferably accomplished using the mathematical algorithm of Karlin and Altschul (1993) Proc. Natl. Acad. Sci USA 90: 5873-5877. Such an algorithm is e.g. incorporated into the BLASTp program of Altschul et al. (1990) J. MoI. Biol. 215: 403-410 available at NCBI (http://www.ncbi.nlm.nih.gov/blast/Blast.cge).
The determination of percent identity is preferably performed with the standard parameters of the BLASTp program.

For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension:1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked. Most prefeably, all parameters are set as outlined above. The term "subject" as used herein refers to a human or an animal, preferably a mammal such as e.g. non-human primates, mice, rats, rabbits, guinea pigs, dogs, cats, cattle, horses, sheep, pigs, goats and the like. Preferably a "subject" in the context of the present invention is a human.

"LRP1 receptor" and "alpha-2-macroglobulin (A2M)" mentioned in the context of the present invention are well known in the art. Therefore the average skilled person can easily retrieve the polynucleotide and amino acid sequences of LRP1 receptor and alpha-2-macroglobulin and orthologous and splice isoforms thereof from any suitable public database such as e.g. the NCBI database (e.g. http://www.ncbi.nlm.nih.gov/pubmed/).

"LRP1 receptor" and "alpha-2-macroglobulin (A2M)" as mentioned in the context of the present invention are preferably mammalian LRP1 receptor and alpha-2-macroglobulin, most preferably human LRP1 and human alpha-2-macroglobulin. Human LRP1 can e.g. be found in the NCBI database under accession number NM_002332.2. Human alpha-2-macroglobulin can e.g. be found in the NCBI database under accession number NM_000014.4.

The LRP1 receptor is sometimes alternatively referred to as CD91.

The term "Wnt signaling pathway" as used herein refers to the Wnt/ß-catenin signaling pathway, the Wnt/Ca2+ signaling pathway and/or the Wnt/planar cell polarity signaling pathway. In a particularly preferred embodiment the Wnt signaling pathway as mentioned in the context of the present invention is the Wnt/ß-catenin signaling pathway, also referred to as the canonical Wnt signaling pathway.

The inventors of the present invention surprisingly found that binding of activated alpha-2-macroglobulin (also referred herein as A2M*) to human LRP1 receptor suppresses specific phenotypes of tumor cells and results in an inhibition of the Wnt/ß-catenin signaling pathway. The inventors of the present invention thus found that the binding of activated alpha-2-macroglobulin to the receptor does not only trigger receptor-mediated endocytosis but that A2M* is also an LRP1 receptor agonist.The inventors further surprisingly found that low doses (such as e.g. a concentration of between 0.01 µM and 0.5 µM) of A2M* significantly up-regulate cellular levels of LRP1 mRNA and protein.

Therefore, in one aspect the present invention relates to a pharmaceutical composition comprising an LRP1 receptor agonist for use in the treatment of cancer as claimed in independent claim 1.

Pharmaceutical compositions for use according to the present invention may be prepared according to any method known to the art for the manufacture of pharmaceutical compositions.

For the production of pills, tablets, sugar-coated tablets and hard gelatin capsules for example, lactose, starch, for example maize starch, or starch derivatives, talc, stearic acid or its salts, etc may be used. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiological sodium chloride solution, alcohols such as ethanol, glycerol, polyols, sucrose, invert sugar, glucose, mannitol, vegetable oils, etc.

In some embodiments the pharmaceutical compositions can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

Examples of suitable excipients for the various different forms of pharmaceutical compositions described herein may e.g. be found in the "Handbook of Pharmaceutical Excipients", 2nd Edition, (1994), Edited by A Wade and PJ Weller.

A pharmaceutical composition for use according to the invention may be administered orally, for example in the form of pills, tablets, lacquered tablets, sugar-coated tablets, granules, hard or soft gelatin capsules, aqueous, alcoholic or oily solutions, syrups, emulsions or suspensions. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures. In some embodiments the pharmaceutical composition for use according to the invention may also be administered rectally, for example in the form of suppositories.

In a preferred embodiment a pharmaceutical composition for use according to the invention is administered parenterally or orally.

In a preferred embodiment a pharmaceutical composition for use according to the invention is suitable for treating a mammal in need thereof. In a particularly preferred embodiment pharmaceutical composition for use according to the invention is suitable for treating a human, in particular a human suffering from cancer or from an HIV infection. In one preferred embodiment, if the subject suffers from an HIV infection it is prefered that the administration dosage is sufficiently large to result in a plasma concentration of between 0.01 µM and 5 µM of said LRP1 receptor agonist as described herein.

The phrase "LRP1 receptor agonist" as used herein in general refers to any compound that is capable of activating the LRP1 receptor.

In one embodiment a molecule is considered to "activate" the LRP1 receptor and thus is considered to be an LRP 1 receptor agonist if it directly or indirectly binds to and activates the LRP1 receptor. Even more preferably such an agonist by directly or indirectly binding to and activating the LRP1 receptor downregulates or inhibits the Wnt signaling pathway in a cell, preferably a cancer cell. Such molecules which directly bind to the LRP1 receptor, such as e.g. activated alpha-2-macroglobulin are particularly preferred.

In another embodiment a molecule is considered to "activate" the LRP1 receptor and thus is considered to be an LRP1 receptor agonist if it either directly or indirectly increases expression of the LRP1 receptor and thus the activity of said receptor. In one preferred embodiment such a molecule is capable of binding to the LRP1 receptor.

In one preferred embodiment the LRP1 agonist for use according to the invention is capable of increasing the expression of LRP1 protein in a cell by at least 10%, at least 20%, at least 30%, at least 40%, at least 50%, at least 60%, at least 70%, more preferably at least 80%, at least 90%, at least 91%, at least 92%, at least 93%, at least 94%, at least 95%, at least 96%, at least 97%, at least 98% or at least 99% when compared to a control cell that is not contacted with any LRP1 receptor agonist. The average skilled person may e.g. determine the amount of protein in a cell without undue burden by quantitative biochemical assays, including e.g. pull down, quantitative Western Blot or any other quantitative assay for determining protein amounts in a cell as are well known in the art.

If said LRP1 receptor agonist is used for the treatment of cancer as listed in independent claim 1, wherein at least part or all of the cancer cells are characterized by an up-regulated or constitutively active Wnt signaling pathway, then it is preferred that the LRP1 receptor agonist by directly or indirectly binding to the LRP 1 receptor downregulates or inhibits the Wnt signaling pathway in a cancer cell. In addition, an LRP1 receptor in such cases may also directly or indirectly increase expression of the LRP1 receptor.

If said LRP1 receptor agonist is used for the treatment of an HIV infection, then it is preferred that the LRP1 receptor agonist is capable of directly or indirectly increasing the expression of the LRP1 receptor.

An "LRP1 receptor agonist" may also be a compound which in its native, i.e. in its non-activated form, does not bind to the LRP1 receptor, but which is transformed or activated inside the body of a subject, such that it is capable of binding to human LRP1 receptor. An example of such an LRP1 receptor agonist is native alpha-2-macroglobulin, which is transformed to activated alpha-2-macroglobulin inside the body of a subject.

Whether a compound is capable of binding to the LRP1 receptor may e.g. be determined by yeast two-hybrid assay or a biochemical assay such as e.g. a pull-down assay, a co-immunoprecipitation assay, an enzyme-linked immunosorbent assay (ELISA), a fluorescence-activated cell sorting (FACS)-based assay or a Plasmon resonance assay. When using pull-down or Plasmon resonance assays, it is useful to fuse at least one of the proteins to an affinity tag such as HIS-tag, GST-tag or other, as is well known in the art of biochemistry.

The average skilled person can without undue burden measure the activity of the Wnt signaling pathway by using e.g. the *Topflash*/*Fopflash* luciferase assay, which is well known in the art and which is e.g. described in Korinek, V., N. Barker, P. J. Morin, D. van Wichen, R. de Weger, K. W. Kinzler, B. Vogelstein, and H. Clevers. 1997, Constitutive transcriptional activation by a β-catenin-Tcf complex in APC-/- colon carcinoma Sci. 275:1784-1787. A TCF reporter plasmid kit comprising the TOPflash and FOPflash reporter plasmids is commercially available e.g. from Millipore. Luciferase activity may e.g. be measured using the Dual-luciferase reporter assay system available from Promega. The TCF reporter plasmid kit and the Dual-luciferase reporter assay system may be used according to the manufacturer's instructions. A synthetic *Renilla* luciferase reporter, e.g. phRG-TK, which is commercially available, e.g. from Promega, may be used as a luciferase internal standard.

For example, cells in which the Wnt signaling activity is to be determined may be transiently transfected with TOPflash or as a control with the FOPflash reporter plasmids and phRG-TK (Promega). Luciferase activity may e.g. be measured using the Dual-luciferase reporter assay system (Promega) and *Renilla* luciferase activity as an internal control.

In another example, the *Topflash*/*Fopflash* luciferase assay may also be performed as described in Examples 1 and 5 herein below.

In order to determine whether a compound, e.g. a potential LRP1 receptor agonist, is capable of downregulating or inhibiting the Wnt signaling pathway, cells, e.g. cancer cells, transfected with the TOPflash reporter plasmid and expressing the LRP1 receptor may be contacted with said compound and luciferase activity may be determined. The measured luciferase activity may then be compared to luciferase activity in control cells that have not been contacted with said compound, wherein a reduction in luciferase activity in the cells treated with the compound versus luciferase activity in control cells indicates that the compound in question is capable of downregulating or inhibiting the Wnt signaling pathway.

The functionality of the Wnt signaling pathway may also be determined using LiCl₂ and inhibition can be analysed by relocation of β-catenin to the plasma membrane as described herein below in Example 6.

Alternatively or additionally any further methods which are suited to determine the activitiy of the Wnt signaling pathway and which are comprised in the art and known to the average skilled person may be used. For example, the expression of Wnt-signaling pathway target genes, such as e.g. TCF1 or LEF1, or other key genes of the Wnt signaling pathway in a cell, e.g. a cancer cell, treated with a compound, e.g. a potential LRP1 receptor agonist, may be compared to a control cell not treated with said compound (e.g. by Western Blotting or real time quantitative PCR), wherein a lower gene expression in the cell treated with the compound versus the control cell indicates that the compound is capable of inhibiting the Wnt signaling pathway. In another example, the levels of β-catenin protein in a cell, e.g. a cancer cell, treated with said compound may be compared to a control cell not treated with said compound, wherein a lower level of the β-catenin protein in the cell treated with the compound versus the control cell indicates that the compound is capable of inhibiting the Wnt signaling pathway.

In one preferred embodiment an LRP1 agonist according to the invention is capable of downregulating or inhibiting the Wnt signaling pathway in a cell by at least 10%, at least 20%, at least 30%, at least 40%, at least 50%, at least 60%, at least 70%, more preferably at least 80%, at least 90%, at least 91%, at least 92%, at least 93%, at least 94%, at least 95%, at least 96%, at least 97%, at least 98% or at least 99% when compared to a control cell that is not contacted with any LRP 1 receptor agonist, or with a control cell which lacks the LRP1 receptor, e.g. a cell which has been treated with siRNA to deplete LRP1 receptor mRNA. In one embodiment the LRP1 receptor is capable of inhibiting the Wnt signaling pathway by 100% when compared to a control cell as mentioned before.

In the context of the present invention a cell "characterized by an up-regulated Wnt signaling pathway" is a cell, preferably a cancer cell, in which the activity of the Wnt signaling pathway is higher than in a control cell. Preferably a control cell is a non-cancerogenous cell. In a preferred embodiment the control cell is from the same tissue as the cell (e.g. the cancer cell) characterized by an "up-regulated Wnt signaling pathway". In another preferred embodiment the control cell is derived from the same source as the cell (e.g. the cancer cell) characterized by an "up-regulated Wnt signaling pathway".

For example, the activity of the Wnt signaling pathway in a cancer cell may be compared to the activity of the Wnt signaling pathway in a non-cancerogenous cell. If the activity of the Wnt signaling pathway is higher in the cancer cell than in the control cell, the Wnt signaling pathway in the cancer cell is up-regulated. In some cases the Wnt signaling pathway may be inactive in the control cell and active in the cancer cell.

In one preferred embodiment, a cancer cell characterized by an up-regulated or constitutively active Wnt signaling pathway exhibits features of malignancy due to an increased expression of Wnt signaling target genes. Expression levels of Wnt signaling target genes in a cancer cell may e.g. be determined as described herein below.

The Wnt signaling pathway in a cell, preferably a cancer cell, may be upregulated by at least 10%, at least 20%, at least 30%, at least 40%, 50%, at least 60%, at least 70%, more preferably at least 80%, at least 90%, at least 91%, at least 92%, at least 93%, at least 94%, at least 95%, at least 96%, at least 97%, at least 98% or at least 99% when compared to a control cell.

Alternatively or additionally, in order to determine whether the Wnt signaling pathway is up-regulated or constitutively active in a cell, preferably a cancer cell, the skilled person may also determine the expression levels of Wnt signaling target genes, preferably human Wnt signaling target genes, such as e.g. c-myc, PPARdelta, uPAR, c-jun, MMP-7, Ahr, Nkd2, Axin2, cyclin D1, CD44, Survivin, Dickkopf, Sox9, Gremlin, Fibronectin 1 and others, in said cell (see e.g.: http://www.stanford.edu/∼musse/pathways/targets.html). Expression levels of Wnt signaling target genes in a cancer cell may e.g. be determined by Western blot analysis or by real time quantitative PCR and compared to the expression levels of the same genes in a control cell, preferably a non-cancerous cell, wherein a higher level of Wnt signaling target gene expression in the cancer cell (i.e.Wnt signaling target gene expression is elevated in the cancer cell) compared to the control cell indicates that the Wnt signaling pathway is up-regulated or constitutively active in the cancer cell. In one preferred embodiment the expression level of a Wnt signaling target gene, preferably a human Wnt signaling target gene, is elevated in the cancer cells characterized by an up-regulated or constitutively active Wnt signaling pathway as described herein. In one preferred embodiment said Wnt signaling target gene is Axin2 (also referred to as conductin), preferably human Axin2 (e.g. NCBI database; accession number NM_004655.3). In another preferred embodiment said Wnt signaling target gene is c-myc, preferably human c-myc.

Expression levels of Wnt signaling target genes may e.g. be at least 10%, at least 20%, at least 30%, at least 40%, at least 50%, at least 60%, at least 70%, more preferably at least 80%, at least 90%, at least 91%, at least 92%, at least 93%, at least 94%, at least 95%, at least 96%, at least 97%, at least 98% or at least 99% higher (elevated) in a cancer cell when compared to expression levels of the same genes in a control cell.

The term "constitutively active" is well known in the art. In the context of the present invention the phrase "constitutively active Wnt signaling pathway" refers to a condition, where the Wnt signaling pathway in a cell, preferably a cancer cell, is permanently active. Such a condition is frequently caused by mutation in one of the Wnt signaling genes. Whether the Wnt signaling pathway is up-regulated or constitutively active in a cell, e.g. a cancer cell, may e.g. be determined by using the *Topflash*/*Fopflash* luciferase assay as described herein before.

According to the invention the pharmaceutical composition is for use in the treatment of cancer selected from the group of cancers claimed in independent claim 1.

The inventors of the present invention have *inter alia* surprisingly found that low doses of A2M* significantly up-regulate cellular levels of LRP1 mRNA and protein.

The present invention in a further aspect thus relates to a pharmaceutical composition comprising an LRP1 receptor agonist for use in the treatment of an HIV infection as claimed in independent claim 2.

As used herein, the term "HIV infection" generally encompasses infection of a subject, preferably a mammalian subject, more preferably a human subject, by the human immunodeficiency virus (HIV) family of retroviruses including e.g. HIV I, HIV II and HIV III (also known as HTLV-II, LAV-1, LAV-2).

The pharmaceutical composition for use in the treatment of an HIV infection according to the invention may be used for the treatment of an HIV infection in a subject, preferably a human subject, who is a carrier of any of the HIV family of retroviruses or a subject, preferably a human subject, who is diagnosed of active AIDS, as well as for the treatment or prophylaxis of the AIDS-related conditions in such subjects. The pharmaceutical composition for use in the treatment of an HIV infection according to the invention may be applied to a subject who is at any one of the several stages of HIV infection progression, which, for example, include acute primary infection syndrome, asymptomatic infection and AIDS.

A subject carrying HIV may be identified by any methods known in the art. For example, a subject infected by HIV can be identified on the basis that the subject is positive for anti-HIV antibody and/or exhibits symptoms of AIDS.

In the present invention, the LRP1 receptor agonist is selected from the group consisting of native or activated alpha-2-macroglobulin, a functional derivative or a functional fragment thereof and an antibody capable of binding to LRP1 receptor.

The term "native alpha-2-macroglobulin" as used herein refers to untransformed, i.e. non-activated alpha-2-macroglobulin.

Native alpha-2-macroglobulin will be transformed inside the body of a subject, to which the pharmaceutical composition according to the invention is applied, to its activated form (A2M*) which is capable of binding to the LRP1 receptor.

In a preferred embodiment the alpha-2-macroglobulin is activated alpha-2-macroglobulin (A2M*).

The term "activated alpha-2-macroglobulin" or "A2M*" is used herein according to its well known meaning in the art. Reference can e.g. be made to Borth W., Alpha 2-macroglobulin, a multifunctional binding protein with targeting characteristics, FASEB J. 1992 Dec;6(15):3345-53. In general the term "activated alpha-2-macroglobulin" or "A2M*" refers to forms of alpha-2-macroglobulin which are capable of binding to LRP1 receptor, i.e. LRP1 receptor-recognized forms of alpha-2-macroglobulin. Alpha-2-macroglobulin may e.g. be activated by binding proteases or small amines, such as e.g. methylamine. Therefore, in one preferred embodiment alpha-2-macroglobulin" or "A2M*" is protease-activated alpha-2-macroglobulin. In another preferred embodiment activated alpha-2-macroglobulin is amine-activated alpha-2-macroglobulin, most preferably methylamine-activated alpha-2-macroglobulin. Other small reactive compounds which disrupt the internal thioester of native, i.e. non-activated alpha-2-macroglobulin, may also be useful for activating alpha-2-macroglobulin. Activated alpha-2-macroglobulin may also be referred to herein as transformed alpha-2-macroglobulin.

Native or activated alpha-2-macroglobulin may e.g. be purified from a sample from a subject. In a preferred embodiment said sample is a plasma sample. In a particularly preferred embodiment said sample is a human plasma sample. Said sample from a subject is preferably separated from the body of the subject.

Activation of alpha-2-macroglobulin may also be achieved ex vivo, preferably by contacting native alpha-2-macroglobulin with proteases, such as e.g. chymotrypsin, or small amines, such as e.g. methylamine.

Native alpha-2-macroglobulin may also be produced recombinantly. For example, a nucleic acid sequences encoding alpha-2-macroglobulin may be cloned into an expression vector and alpha-2-macroglobulin may be expressed in a host cell, preferably a mammalian host cell, and subsequently purified. If desired, purified alpha-2-macroglobulin may then be activated in vitro as described above. The skilled person will be familiar with recombinant protein expression techniques and suitable expression vectors and host cells will be known to the skilled person.

Pharmaceutical compositions for use according to the invention which comprise native or activated alpha-2-macroglobulin or a functional derivative or a functional fragment thereof provide inter alia the advantage that alpha-2-macroglobulin and activated alpha-2-macroglobulin is produced naturally in the body and is therefore non-toxic. Furthermore, alpha-2-macroglobulin can easily be generated, e.g. by purifying the protein from a human plasma sample (see e.g. J Pharmacol Exp Ther. 2006 Aug;318(2):762-71).

The term "functional derivative" as used herein refers to a modified version of native or activated alpha-2-macroglobulin as defined in independent claims 1, 2 and 4, namely that one or more amino acids of the polypeptide may be deleted, inserted, modified and/or substituted. The derivative is functional if it is capable of activating the LRP1 receptor. Preferably, a functional derivative is capable of binding to the LRP1 receptor or can be transformed inside the body of a subject, preferably a human, to a form which is capable of binding to the LRP1 receptor. Binding of the derivative to LRP1 receptor preferably triggers downregulation of the Wnt-signaling pathway in the cell. In one embodiment a functional derivative either directly or indirectly increases expression of the LRP1 receptor.

In one preferred embodiment a functional derivative does not comprise more than 1, 2, 3, 4, 5, 6, 7, 8, 9, 10, 15, 20, 25, 30, 35, 40, 45, 50, 60, 70, 80, 90 or more than 100 amino acid changes (i.e. deleted, inserted, modified and/or substituted amino acids). In another preferred embodiment not more than 1%, 2%, 3%, 4%, 5%, 6%, 7%, 8%, 9%, 10%, 15%, or more than 20%, most preferably not more than 5% of all amino acids of the polypeptide are changed (i.e. are deleted, inserted, modified and/or substituted amino acids).

Amino acids of the polypeptide may also be modified, e.g. chemically modified. A derivative is a protein, where the side chain or a free amino or carboxy-terminus of an amino acid of the protein is modified by e.g. glycosylation, amidation, phosphorylation, ubiquitination, etc.. A substitution in a derivative may be a conservative or a non-conservative substitution, preferably a conservative substitution. In some embodiments, a substitution may also include the exchange of a naturally occurring amino acid with a not naturally occurring amino acid. A conservative substitution comprises the substitution of an amino acid with another amino acid having a chemical property similar to the amino acid that is substituted.

A "functional derivative" shows at least 80%, at least 81%, at least 82%, at least 83%, at least 84%, at least 85%, at least 86%, at least 87%, at least 88% at least 89%, more preferably at least 90%, even more preferably at least 91%, at least 92%, at least 93%, at least 94% least 95%, at least 96%, at least 97% or at least 98% and most preferably at least 99% sequence identity over its entire length to the amino acid sequence it refers to.

The term "functional fragment" as used herein refers to a portion of native or activated alpha-2-macroglobulin, which is capable of activating the LRP1 receptor. Preferably, said functional fragment is capable of binding to the LRP1 receptor or can be transformed inside the body of a subject, preferably a human, to a form which is capable of binding to the LRP1 receptor and which upon binding the LRP1 receptor triggers down-regulation of the Wnt-signaling pathway in the cell. In one embodiment a functional fragment either directly or indirectly increases expression of the LRP1 receptor. In a preferred embodiment a functional fragment has a length of between 40 to 400 amino acids, more preferably between 80 and 350 amino acids, even more preferably a functional fragment has a length of between 100 and 200 amino acids.

In a preferred embodiment the functional fragment comprises or consists of the LRP1 receptor binding-domain of alpha-2-macroglobulin, preferably human alpha-2-macroglobulin.

The LRP1 receptor binding domain of human alpha-2-macroglobulin preferably corresponds to residues 1314 to 1451 of the full length human alpha-2-macroglobulin, e.g. full length human alpha-2-macroglobulin according to NCBI accession numberNM_000014. The functional fragment in some embodiments may also comprise or consists of the LRP1 receptor binding-domain of alpha-2-macroglobulin, preferably human alpha-2-macroglobulin, wherein one or more amino acids of the LRP1 receptor binding-domain are deleted, inserted, modified and/or substituted, provided that said LRP1 receptor binding-domain is still capable of activating the LRP1 receptor. In such cases, the LRP 1 receptor binding domain preferably does not comprise more than 1, 2, 3, 4, 5, 6, 7, 8, 9, 10, 15, 20, 25, 30, 35, 40, 45, 50 or more than 60 amino acid changes (i.e. deleted, inserted, modified and/or substituted amino acids). In another preferred embodiment not more than 1%, 2%, 3%, 4%, 5%, 6%, 7%, 8%, 9%, 10%, 15%, or more than 20%, most preferably not more than 5% of all amino acids of the LRP1 receptor binding domain are changed (i.e. are deleted, inserted, modified and/or substituted amino acids). Preferably, said LRP1 receptor binding-domain is still capable of binding to the LRP1 receptor.

The functional fragment may also comprise or consist of a portion of the LRP 1 receptor binding domain of alpha-2-macroglobulin, provided that said portion is capable of activating the LRP1 receptor. Preferably, said portion is capable of binding to the LRP1 receptor or can be transformed inside the body of subject to a form which is capable of binding to the LRP1 receptor and which upon binding the LRP1 receptor triggers down-regulation of the Wnt-signaling pathway in the cell. In one embodiment said portion either directly or indirectly increases expression of the LRP1 receptor.

An antibody capable of binding to LRP1 receptor is preferably a monoclonal or polyclonal antibody. The term "antibody" refers to any immunoglobulin protein or portion thereof which is capable of binding to the LRP1 receptor.

In some embodiments, antigen-binding portions may include Fab, Fab', F(ab')2, Fd, Fv, dAb, and complementarity determining region (CDR) variants, single-chain antibodies (scFv), chimeric antibodies, humanized antibodies, diabodies, and polypeptides that contain at least a portion of an antibody that is sufficient to confer specific antigen binding to the LRP1 receptor.

In some embodiments the antibody may also be selected from antibody variants or fragments that are sufficient to confer binding to the LRP1 receptor such as e.g. single chain antibodies, diabodies, minibodies, single chain Fv fragments (sc(Fv)), sc(Fv)₂ antibodies, Fab fragments or a F(ab')₂ fragments and polypeptides that contain at least a portion of an antibody that is sufficient to confer binding to the LRP1 receptor.

Antibodies may be produced according to any suitable method known to the person skilled in the art. Polyclonal antibodies may e.g. be produced by immunization of animals with the antigen of choice, whereas monoclonal antibodies of defined specificity may e.g. be produced using the hybridoma technology developed by Köhler and Milstein (Köhler and Milstein, 1976, Eur. J. Immunol., 6:511-519).

Whether an antibody, a functional derivative or a functional fragment is capable of binding to the LRP1 receptor may e.g. be determined by yeast two-hybrid assay or a biochemical assay such as e.g. a pull-down assay, a co-immunoprecipitation assays, an enzyme-linked immunosorbent assay (ELISA), a fluorescence-activated cell sorting (FACS)-based assay or a Plasmon resonance assay. When using pull-down or Plasmon resonance assays, it is useful to fuse at least one of the proteins to an affinity tag such as HIS-tag, GST-tag or other, as is well known in the art of biochemistry.

In one preferred embodiment the antibody capable of binding to LRP1 receptor is rabbit anti-LRP1 Ab as described below in the example section (e.g. example 1). If the pharmaceutical composition according to the invention is to be applied to humans, it is preferred that said antibody is humanized.

The term "small molecule" as used herein refers to small organic compounds having low molecular weight. A small molecule may be a synthetic compound not known to occur in nature or a naturally- occurring compound isolated from or known to occur in natural sources, such as e.g. cells, plants, fungi, animals and the like. A small molecule in the context of the present invention preferably has a molecular weight of less than 5000 Dalton, less than 4000 Dalton, less than 3000 Dalton or less than 2000 Dalton.

In another preferred embodiment a small molecule in the context of the present invention has a molecular weight of 50 to 3000 Dalton, preferably of 100 to 2000 Dalton, more preferably of 150 to 1500 Dalton and most preferably of 200 to 1000 Dalton.

In a preferred embodiment the pharmaceutical composition for use according to the invention comprises a further active compound suitable for the treatment of cancer and/or a further active compound suitable for the treatment of an HIV infection.

In one preferred embodiment the further active compound suitable for the treatment of cancer is a chemotherapeutic agent. Chemotherapeutic agents suitable for the treatment of cancer are well known to the skilled person. Examples of chemotherapeutic agents are e.g. temozolomide, adriamycin, doxorubicin, epirubicin, 5-fluorouracil, cytosine arabinoside ("Ara-C"), cyclophosphamide, thiotepa, busulfan, cytoxin, taxoids, e. g., paclitaxel, toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin,mitomycins, melphalan andotherrelated nitrogen mustards and hormonal agents that act to regulate or inhibit hormone action on tumors such as tamoxifen and onapristone.

Preferably, the further active compound suitable for the treatment of an HIV infection is an antiviral, more preferably an antiretroviral compound, such as e.g. an entry inhibitor, including fusion inhibitors and chemokine coreceptor (CCR) inhibitors, a nucleoside/nucleotide reverse transcriptase inhibitor (NRTI), a non-nucleoside reverse transcriptase inhibitor (NNRTI), an integrase inhibitor or a protease inhibitor (PI). Suitable antiretroviral compounds are well known to the skilled person and comprise e.g. Maraviroc, Raltegravir, Etravirine, Saquinavir, Acyclovir, Zidovudine and Tenofovir.

The pharmaceutical composition for use according to the invention may comprise one or more than one further active compound suitable for the treatment of cancer and/or one or more than one further active compound suitable for the treatment of an HIV infection. In a preferred embodiment the pharmaceutical compostion according to the invention comprises 1,2,3,4,5,6,7,8,9,10 or more than 10 further active compounds suitable for the treatment of cancer and/or 1,2,3,4,5,6,7,8,9,10 or more than 10 further active compound suitable for the treatment of an HIV infection.

In other preferred embodiments one or more separate compositions comprising the aforementioned active compounds suitable for the treatment of cancer and/or the aforementioned active compounds suitable for the treatment of an HIV infection may be administered to a subject, preferably a human subject, in combination with a pharmaceutical composition according to the invention comprising an LRP1 receptor agonist.

In a further aspect the present invention relates to the use of an LRP1 receptor agonist or a pharmaceutical composition according to the invention for the manufacture of a medicament as claimed in independent claim 4.

For the use according to the invention the LRP1 receptor agonist is selected from the group consisting of native or activated alpha-2-macroglobulin, a functional derivative or a functional fragment thereof and an antibody capable of binding to LRP1 receptor.

Native alpha-2-macroglobulin will be transformed inside the body of a subject, to which the pharmaceutical application according to the invention is applied, to its activated form (A2M*) which is capable of binding to the LRP1 receptor.

In a preferred embodiment the alpha-2-macroglobulin is activated alpha-2-macroglobulin (A2M*).

For the use according to the invention the cancer is selected from the group listed in independent claim 4.

In another preferred embodiment of the use according to the invention, the cancer cells are malignant cancer cells.

In a further aspect the present invention provides an in vitro method for identifying a therapeutic agent for treating cancer as claimed in independent claim 6.

In a preferred embodiment the compound to be tested is an antibody or a small molecule. Preferably, said small molecule has a molecular weight of between 50 Dalton and 3000 Dalton.

For the method according to the invention said cancer is selected from the group listed in independent claim 6.

For the method according to the invention said at least one cell is a cancer cell, preferably a malignant cancer cell. In a particularly preferred embodiment said at least one cell is an astrocytoma cell or a hematopoetic cancer cell.

In order to determine whether the compound binds to the LRP1 receptor expressed by the at least one cancer cell, the skilled person may e.g. use fluorescently labeled compounds or a biochemical assay such as e.g. a pull-down assay, a co-immunoprecipitation assays, an enzyme-linked immunosorbent assay (ELISA), a fluorescence-activated cell sorting (FACS)-based assay or a Plasmon resonance assay.

Furthermore, binding of the compound to the LRP1 receptor may also be determined functionally, i.e. it may be tested whether the compound is capable of down-regulation or inhibiting the Wnt signaling pathway in the at least one cell.

Whether binding of the compound to the LRP1 receptor results in downregulation or inhibition of the Wnt signaling pathway in the at least one cell may e.g. be determined by using the methods described herein above, e.g. by using the *Topflash*/*Fopflash* luciferase assay or by deteremining the expression levels of Wnt signaling target genes.

In order to determine whether a cell expresses LRP1 receptor on its surface, the skilled person may e.g. perform immunohistochemistry and/or immunofluorescence analyses. For example, labeled antibodies, e.g. fluorescently labeled antibodies, against LRP1 may be applied to the cell and labeling may be detected. In said example, if the cell expresses LRP1 receptor, labeling of the cell surface will be observed. In this context, suitable antibodies are e.g. antibodies that recognize the α chain and/or the β chain of LRP1.

In another aspect the present invention relates to a kit of parts as claimed in independent claim 9 comprising the pharmaceutical composition for use according to the above aspects and diagnostic reagents suitable for determining whether the Wnt-signaling pathway is up-regulated or constitutively active in a cell.

In a preferred embodiment the diagnostic reagents suitable for determining whether the Wnt-signaling pathway is up-regulated or constitutively active in a cell are selected from the group consisting of a Wnt reporter plasmid, a luciferase reporter plasmid and an oligonucleotide primer pair suitable for quantifying the expression of a Wnt signaling pathway target gene by real-time PCR.

In one preferred embodiment the Wnt reporter plasmid is selected from the group consisting of the TOPflash reporter plasmid, the FOPflash reporter plasmid, the TOP-Gau reporter plasmid and the FOP-Gau reporter plasmid (the TOP-Gau reporter plasmid and the FOP-Gau reporter plasmid are herein described below in example 1). In a further preferred embodiment the luciferase reporter plasmid is a *Renilla* luciferase reporter plasmid, preferably phRG-TK (Promega). An oligonucleotide primer pair suitable for quantifying the expression of a Wnt signaling pathway target gene by real-time PCR may be any oligonucleotide primer pair suitable for amplification of any given Wnt signaling target gene that can be used in real time PCR. How to design oligonucleotide primers for real time PCR is well known in the art. Examples of human Wnt signaling target genes are e.g. c-myc, PPARdelta, uPAR, c-jun, MMP-7, Ahr, Nkd2, Axin2, cyclin D1, CD44, Survivin, Dickkopf, Sox9, Gremlin and Fibronectin 1.The kit may contain some or all of the aforementioned diagnostic reagents.

The pharmaceutical composition for use according to the invention comprised in the kit and/or the aforementioned diagnostic reagents may be packaged in containers such as e.g. vials, test tubes, flasks, bottles, syringes or other containers into which a pharmaceutical composition and/or a reagent may be placed. Preferably the reagents are suitably aliquoted. A kit typically also comprise packaging for containing the various components for commercial sale. A kit may also include instructions for employing the kit components.

The present invention will now be described with respect to some of its specific examples.

### EXAMPLES

### Example 1 - Materials and Methods

Anti-human LRP1 mAb (clone II2C7 and II4/8), rabbit polyclonal anti-LRP1 Ab, native A2M, methylamine-transformed A2M* and FITC-A2M* were purchased from BioMac (Leipzig, Germany), WST-1 proliferation kit from Roche (Mannheim, Germany), cellulose nitrate membranes from Whatman Schleicher & Schuell (Dassel, Germany), Dulbecco's modified Eagle's (DMEM) and RPMI-medium, FCS and Cy3-labeled goat anti-mouse Ab from Invitrogen (Karlsruhe, Germany), Growth Factor Reduced Matrigel™ Matrix and polycarbonate mesh, mouse anti-human E-cadherin, N-cadherin and β-catenin from BD Biosciences (Heidelberg, Germany), hematoxylin from Merck (Darmstadt, Germany), trypan blue from Seromed (Berlin, Germany), rabbit anti-human β-actin Ab from Abcam (Cambridge, MA), HRP-labeled goat anti-mouse Ab and Real Detection System Peroxidase/3,3'-diaminobenzidine (DAB) Rabbit/Mouse Kit from Dako (Hamburg, Germany), Protease inhibitor cocktail from Sigma-Aldrich (Taufkirchen, Germany) and chemiluminescence detection kit from Boehringer (Mannheim, Germany). Plasmid *pCMV-GLUC* was obtained from Prolume Nanolight Inc. (Pinetop, AZ), TCF Reporter Plasmid Kit from Millipore (Schwallbach, Germany), TransIT®-LT1 from Mirus Corporation (Madison) and *Gaussia* luciferase transfection kit and coelenterazine from PJK (Kleinbittersdorf, Germany). A2M (clone α1-IIE7) and prostate specific antigen (PSA, clone 1B7) mAbs were used as described previously (1, 2).

**Cell lines.** Human astrocytoma 3121N1 cells (ECACC; 86030102), LNCaP (DSMZ; ACC 256), PC-3 (CRL-1435; ATCC) and DU-145 (DSMZ; ACC 261) were used for this study.

**Cell culture.** Human astrocytoma 1321N1cells were cultured in DMEM, 10% FCS (DMEM-FCS), 100 U penicillin and 100 µg streptomycin/ml at 37°C / 5% CO₂ in a humidified atmosphere. Cell monolayers were dissociated using 0.04% trypsin/0.03% EDTA in 50 mM PBS (pH 7.4). Cellular protein extracts of tumor cells were prepared using extraction buffer (25 mM Tris, 2 mM EDTA, 10% Glycerol, 1% Triton X-100, 2 mM DTT, 1 mM PMSF, pH. 7.0) containing 0.3% protease inhibitor cocktail. Protein content was determined (3) and human fibroblast cultures were set up (4) as previously described.

**Endocytosis of FITC-labeled alpha2-macroglobulin.** Astrocytoma cell cultures (10⁵/2 ml DMEM-FCS/well) were set up and FITC-labeled A2M* (0.1 µM) was added to cavities containing 2 µM A2M*, 2 µM polyclonal anti-LRP1 Ab or rabbit pre-immune Ig (2 µM) and incubated for 30 min to estimate binding/uptake of labeled A2M*. Plates were chilled on ice, washed twice (each with 2 ml PBS), and supernatants were discarded. Cells were removed by trypsination and incubated with extraction buffer in the absence of protease inhibitors. Samples were disintegrated by sonication (3x15 seconds) and centrifugation at 13,000 rpm for 15 min at 4°C. Supernatants were analyzed using PerkinElmer LS50B.

**Immunochemistry.** According to standard protocols, immunochemical staining of astrocytoma 1321N1 cells was performed using the primary anti-human Abs directed against E-cadherin, N-cadherin, LRP1, β-catenin and β-actin. Immunofluorescence detection was accomplished with Cy3-labeled goat anti-mouse Ab. 3-µm paraffin-embedded sections of spheroids were stained with mouse anti-LRP1 mAb (clone II2C7) in combination with a secondary biotinylated Ab and HRP-labeled streptavidin. Stained cells were evaluated with a confocal laser scanning microscope (LSM510 META, Carl Zeiss, Jena, Germany).

**Immunoblotting.** Following cell lysis, 20-100 µg of cell lysate proteins were loaded to 4-20% SDS-PAGE, blotted to membranes. Primary Ab used were rabbit anti-LRP1 (µg/ml) and anti-β-actin Ab (0.5 µg/ml), anti-β-catenin, anti-E-cadherin and anti-N-cadherin mAb (10µg/ml each). Detection was accomplished with HRP-labeled goat anti-mouse (1:1000) or anti-rabbit (1:2000) Ab. Bands were visualized by chemiluminescence detection.

**Proliferation/vitality.** Astrocytoma cells were cultured as described above, putative receptor ligands were added, and cell proliferation was analyzed using the WST-1 assay (5).

**Cell viability test.** The effect of A2M* on viability of astrocytoma cells was inspected by trypan blue exclusion (6).

**LDH release.** LDH activity was assayed in the supernatant of astrocytoma cells cultured for 24 h (5).

**Migration and invasion assay.** Cell movement was evaluated in Boyden chambers using 24-well culture plates equipped with 8-µm pore polycarbonate mesh, the bottom well was filled with 800 µl DMEM-FCS. For invasion analysis the membranes of inserts were coated with 25 µl Growth Factor Reduced Matrigel™ Matrix. Cells (4x10⁴ in 100 µl DMEM, 0.5% FCS) were seeded in the upper compartment to adhere overnight. Test substances were added and incubation was continued for 6 h (migration) or 42 h (invasion). Cells were removed and the membranes were fixed with Karnowski solution, and stained with hematoxylin.

**Anchorage-independent growth.** Six-well plates were coated with 1.5 ml of a base layer of 0.6% basal agar in 2x DMEM-FCS. Cells (8x10⁵/well) were added in a mixture of 0.4% agar and 2x DMEM/20% FCS. Test substances were initially mixed into the upper agar layer. Cultures were evolved for 14 days, and replenished daily with 100 µl DMEM-FCS. Finally, the plates were stained with 0.5 ml of 0.005% Crystal violet, and colonies of > 20 cells were counted.

**Adhesion assay.** Well plates were coated with 0.1% gelatin solution before adding 10⁴ cells/100 µl DMEM-FCS in the absence or presence of A2M*, the receptor-associated protein (RAP) or anti-LRP1 Ab. After certain time lapses, non-adherent cells were washed away and the remaining cells were fixed with 4% para-formaldehyde solution, stained with Crystal violet (1%), washed, and de-stained with acetic acid (33%) followed by reading the absorbance at 590 nm against the blank.

**Tumor spheroid cultures.** Astrocytoma cells (5x10⁶) were transferred into 10 ml DMEM-FCS containing test substances in 50 ml plastic tubes and rolled at 5 rpm at 37°C/ 5% CO₂ for 3 weeks. Spheroid diameters (mean of 2 orthogonal diameters) were determined and medium was changed daily.

**Preparation of Fab-fragments.** Fab fragments of rabbit anti-LRP1 Ab (10 mg) were prepared using the Fab Preparation Kit from Pierce (Rockland, USA).

**Relative gene expression.** Isolation of total RNA of astrocytoma cells exposed to serial concentrations of A2M*, digestion of genomic DNA, reverse transcription and purification of cDNA were performed as previously described (7). For real-time PCR, the following forward (f) and reverse (r) primer sequences were used: (i) *GAPDH* (NM_002046.3): f: 5'-AGCCACATCGCTCAGACAC-3' (SEQ ID NO. 1), r: 5'-GCCCAATACGACCAA ATC C-3'(SEQ ID NO. 2); (ii) *LRP1* (NM_002332.2) f: 5'-GATGAGACACACGCCAACTG-3'(SEQ ID NO. 3), r: 5'-CGGCACTGGAACTCATCA-3'(SEQ ID NO. 4); (iii) *LRP5* (NM_002335.2) f: 5'-GAACATCAAGCGAGCCAAG-3'(SEQ ID NO. 5), r: 5'-TGGCTCAGAGAGGTCAAAACA-3'(SEQ ID NO. 6); (iv) *LRP6* (NM_002336.2) f: 5'-ATCCGAAAGGCACAAGAAGA-3'(SEQ ID NO. 7), r: 5'-GACTCGGAACTGAGCTCACAA-3'(SEQ ID NO. 8); (v) *FZD* (NM_001463.2) f: 5'-TCATGGGCTATGAAGATGAGG-3'(SEQ ID NO. 9), r: 5'-TCATATCCCAGCGCTTAACTT T-3'(SEQ ID NO. 10); (vi) *Wnt1* (NM_005430.2) f: 5'-CGCTGGAACTGTCCCACT-3'(SEQ ID NO. 11), r: 5'-AACGCCGTTTCTCGACAG-3'(SEQ ID NO. 12); (vii) *Wnt3A* (NM_033131.2) f: 5'-AACTGCACCACCGTCCAC-3'(SEQ ID NO. 13), r: 5'-CCGACTCCCTGGTAGCTTT-3'(SEQ ID NO. 14); (viii) *Wnt5A* (ENST00000264634.4) f: 5'-TAAGCCCAGGAGTTGCTTTG-3'(SEQ ID NO. 15), r. 5'-CTGAACAGGGTTATTCATACCTAGC-3') (SEQ ID NO. 16); (ix) *Wnt10B* (U81787.1) f: 5'-GCGAATCCACAACAACAGG-3'(SEQ ID NO. 17), r: 5'-TCCAGCATGTCTTGAACTGG-3'(SEQ ID NO. 18); (x)*, β-catenin* (X87838.1) f: 5'-GCTTTCAGTTGAGCTGACCA-3'(SEQ ID NO. 19), r: 5'-AAGTCCAAGATCAGCAGTCTC A-3'(SEQ ID NO. 20); (xi) *E-cadherin* (AB025105.1) f: 5'-CCCGGGACAACGTTTATTAC-3'(SEQ ID NO. 21), r: 5'-GCTGGCTCAAGTCAAAGTCC-3' (SEQ ID NO. 22) and *N-cadherin* (M34064.1) f: 5'-GGTGGAGGAGAAGAAGACCAG-3'(SEQ ID NO. 23), r: 5'-GGCATCAGGCTCCACAGT-3'(SEQ ID NO. 24). Reactions started by an initial activation step for 10 min at 95°C, and each following cycle consisted of a denaturation step for 15 s at 94 °C, annealing for 5 s at 60°C and extension for 15 s at 72°C. GAPDH was chosen as a reference house-keeping gene as it showed amplification efficiency similar to those of other cytokine genes. REST© software (8) was used to estimate gene expression relative to controls.

**TCF-Reporter Assay.** The reporter gene *TOP-Gau* containing a secreted luciferase of *Gaussia princeps,* controlled by two sets of three copies of the TCF binding site (wild type) and a thymidine kinase minimal promoter, was derived by combining the reporter gene *TOP-flash* (TCF Reporter Plasmid Kit) with the luciferase gene. The luciferase coding region was derived from the plasmid *pCMV-GLUC.* The mutated *FOP-Gau* reporter gene (with two full and one incomplete copy of the TCF binding site followed by three copies in the reverse orientation) was derived from *FOP-flash* and *pCMV-GLUC.*

Cells, seeded at 10⁵ per 1ml medium, were transfected with 1 µg DNA using TransIT®-LT1, and then incubated with test substances for 36 h. *Gaussia* luciferase activity was assessed in 10 µl cell supernatants using 50 µl assay reagent on Orion-II microplate luminometer.

**Statistical analysis.** Statistics were done using Wilcoxon's rank-sum test, and PCR results were analyzed using pair-wise fixed reallocation randomization test incorporated in REST© software; significance were considered at p< 0.05. Otherwise else indicated, all data indicate means±SD of three independent experiments.

### Example 2 - LRP1 is highly expressed in astrocytoma cells and mediates endocytosis of A2M*.

LRP1 is highly expressed in 1321N1 astrocytoma cells compared with two prostate cancer cell lines (LNCaP and DU-145) or fibroblasts (Fig. 1A). FITC-labeled A2M* was rapidly endocytosed by tumor cells; its uptake is diminished by adding non-labeled A2M* or anti-LRP1 Ab (Fig. 1B) highlighting the ability of the polyclonal Ab to block the A2M* binding site of LRP 1.

### Example 3 - A2M* inhibits the growth factor-elicited cell proliferation.

A2M* strongly inhibited proliferation of tumor cells under optimal growth-promoting conditions, whereas native A2M exerted only marginal inhibitory effects (Fig. 2A). To explore an alternative pathway other than neutralizing growth factors (9, 10), the cellular receptor was blocked with a polyclonal Ab that does not mediate clearance of growth factors. As depicted in figure 2A, anti-LRP1 Ab exhibited a similar influence on cell proliferation compared with A2M*, further indicating LRP1 activation by A2M*. The polyclonal Ab fraction presumably contained mAb(s) that specifically reacted with the A2M* binding domain of LRP1 and can mimic the binding of A2M* to the receptor.

Inhibition of cell proliferation by A2M* was not due to cell toxicity as indicated by the unaltered LDH release (Fig. 2B). Moreover, the average cell vitality was 95±3%, 93±3.7% and 96±8% for control, 0.2 µM A2M*- and 0.2 µM A2M-treated cells, respectively.

### Example 4 - A2M* inhibits migration, adhesion and invasion of 1321N1 astrocytoma cells.

Migration rate of 1321N1 cells was inversely correlated with the concentration of A2M*. Cell migration was only slightly changed by native A2M (Fig. 3A). The specificity of A2M*-LRP1 interaction was confirmed by applying mAb (α1; clone α1-IIE7) known to react with the C-terminal receptor-binding domain of A2M* (1), its presence abrogated the inhibiting effect of A2M*, probably through blocking the binding of A2M* to LRP1. RAP, known to abolish binding of almost all ligands to LRP1 (11), diminished cell migration, similar to the polyclonal anti-LRP1 Ab. As controls, mAbs against α (II2C7) and β (II48) chains of LRP1 were used as well as an irrelevant Ab against PSA (1B7). All of these mAb showed insignificant effect on cell migration.

Because A2M* and polyclonal anti-LRP1 Abs inhibited cell proliferation and migration, it was hypothesized that inhibition may be due to LRP1-dependent interference with malignant cell adhesion factor(s). Results depicted in figure 3B showed that, in comparison to controls, the adhesion of tumor cells was significantly reduced after 120 min in the presence of 0.2 µM A2M*, 1 µM RAP or 1 µM polyclonal anti-LRP1. No difference between control and ligand-treated cells was observed following 200-min incubation. These data indicate that LRP1 might be involved in cell adhesion, and receptor ligands can diminish cell-matrix interaction. Tumor cells invade extracellular matrix either through protease-dependent processes or amoeboid movement (12). Therefore, inhibiting extracellular proteases would eventually reduce the ability of cells to invade the matrix network. To prove this, a cell invasion assay was conducted to allow cells to migrate through Matrigel-coated 8-µm pore invasion chambers. In the presence of protease inhibitors, the invasion of tumor cells was reduced by approximately 50% relative to controls. Similar results were obtained by A2M*, polyclonal anti-LRP1 Ab or RAP (Fig. 3C). The LRP1 mAb (clone II2C7) and the irrelevant anti-PSA mAb (1B7) failed to inhibit cell invasion. By applying poly- and monovalent anti-LRP1 Ab obtained by papain digestion it was corroborated that the effect of A2M* on cell invasion is brought about by direct activation of LRP1.

### Example 5-A2M* or anti-LRP1 antibody inhibits growth of spheroids and anchorage-independent growth of 1321N1 astrocytoma cells.

Tumor spheroids provide a suitable three-dimensional *in vitro* model that may mimic the cellular environment of a poorly-vascularized, hypoxic solid tumor (13). The growth of approximately 50 to 100 spheroids per group was monitored to study the size distribution over time. The initial aggregation of cells (over 3 days) showed cell clumps of 40-120-µm diameter (Fig. 4A). The size of the spheroids increased progressively with time but size distribution became more heterogeneous due to shedding of tumor cells from surface and formation of sub-spheroids (Fig. 4B, left). Immunostaining of LRP1 (Fig. 4B, right) indicated its presence at the margin, which may constitute the proliferative zone of a spheroid. Results shown in Fig. 4C revealed that spheroids' diameter increased steadily up to day 6, then followed by a plateau phase till day 14. Compared to controls, A2M* (0.2 µM) or anti-LRP1 Ab (1µM) inhibited the growth of tumor spheroids. A2M* was more potent at the initial phase of spheroid accretion comparable to anti-LRP1 Ab. This indicates the implication of LRP1 in cell-cell adherence and that ligation of the A2M* binding domain of LRP1 modulates this process.

Furthermore, the effect of A2M* and Abs on anchorage-independent growth that is known to be a good index of tumorigenicity was investigated. Astrocytoma cells were found to form colonies in the soft-agar assay (Fig. 4D, left) and A2M* (0.2 µM) significantly inhibited colony formation by more than 50%. Compared to the control, polyclonal anti-LRP1 Ab elicited a similar effect (Fig. 4D, right).

### Example 6- A2M* mitigates the Wnt/β-catenin pathway in 1321N1 astrocytoma cells and increases expression of LRP1

Progression of human tumors is often associated with the induction of the canonical Wnt/β-catenin signaling. Using the *Topflash*/*Fopflash* assay, it was found that the Wnt/β-catenin signaling is constitutively active in 1321N1 cells discernible at the high activity of the *Topflash* versus *Fopflash* signal (Fig. 5A, left); the latter used as internal control. The functionality of the Wnt/β-catenin signaling cascade was demonstrated by adding 5 mM LiCl₂ that is known to inhibit glycogen synthase kinase (GSK)-3β leading to stabilization of β-catenin, which then accumulates around and translocates into the nucleus. This effect was notably abated in the presence of 0.1 µM A2M*, and β-catenin relocated to the plasma membrane (PM) (Fig. 5A, middle panel) implying inhibition of the canonical Wnt/β-catenin pathway by A2M* (Fig. 5A, right). This was additionally affirmed by evaluating the expression of various genes (Fig. 5B). Low doses of A2M* significantly up-regulated FZD, LRP1 and E- and N-cadherin at mRNA and protein level, and slightly up-regulated β-catenin levels (Fig. 5C); LRP5 and LRP6 mRNA remained unchanged. The expression of Wnt1, Wnt5A and Wnt10B mRNA was down-regulated (Fig. 5B), while Wnt3A mRNA was not detected. Anti-LRP1 Ab exerted comparable effects.

As shown in figure 5D, A2M* increased cellular level of LRP1 and induced the relocation of β-catenin to the PM. In the presence of A2M*, the size of the cytosolic compartments and nuclei diminished in a dose-dependent fashion (middle panel).

### LITERATURE

1. Birkenmeier, G. and Stigbrand, T. Production of conformation-specific monoclonal antibodies against alpha 2 macroglobulin and their use for quantitation of total and transformed alpha 2 macroglobulin in human blood. J Immunol Methods, 162: 59-67, 1993.
2. Baumgart, Y., Otto, A., Schafer, A., Usbeck, E., Cott, C., Schott, A., Tornack, M., Wenzel, A., Mossie, A., and Birkenmeier, G. Characterization of novel monoclonal antibodies for prostate-specific antigen (PSA) with potency to recognize PSA bound to alpha 2-macroglobulin. Clin Chem, 51: 84-92, 2005.
3. Bradford, M. M. A rapid and sensitive method for the quantitation of microgram quantities of protein utilizing the principle of protein-dye binding. Anal Biochem, 72: 248-254, 1976.
4. Birkenmeier, G., Heidrich, K., Glaser, C., Handschug, K., Fabricius, E. M., Frank, R., and Reissig, D. Different expression of the alpha2-macroglobulin receptor/low-density lipoprotein receptor-related protein in human keratinocytes and fibroblasts. Arch Dermatol Res, 290: 561-568, 1998.
5. Santel, T., Pflug, G., Hemdan, N. Y., Schafer, A., Hollenbach, M., Buchold, M., Hintersdorf, A., Lindner, I., Otto, A., Bigl, M., Oerlecke, I., Hutschenreuter, A., Sack, U., Huse, K., Groth, M., Birkemeyer, C., Schellenberger, W., Gebhardt, R., Platzer, M., Weiss, T., Vijayalakshmi, M. A., Kruger, M., and Birkenmeier, G. Curcumin inhibits glyoxalase 1: a possible link to its anti-inflammatory and anti-tumor activity. PLoS ONE, 3: e3508, 2008.
6. Hollenbach, M., Hintersdorf, A., Huse, K., Sack, U., Bigl, M., Groth, M., Santel, T., Buchold, M., Lindner, I., Otto, A., Sicker, D., Schellenberger, W., Almendinger, J., Pustowoit, B., Birkemeyer, C., Platzer, M., Oerlecke, I., Hemdan, N., and Birkenmeier, G. Ethyl pyruvate and ethyl lactate down-regulate the production of pro-inflammatory cytokines and modulate expression of immune receptors. Biochem Pharmacol, 76: 631-644, 2008.
7. Hemdan, N. Y. The role of interleukin-12 in the heavy metal-elicited immunomodulation: relevance of various evaluation methods. J Occup Med Toxicol, 3: 25, 2008.
8. Pfaffl, M. W., Horgan, G. W., and Dempfle, L. Relative expression software tool (REST) for group-wise comparison and statistical analysis of relative expression results in real-time PCR. Nucleic Acids Res, 30: e36, 2002.
9. Chu, C. T. and Pizzo, S. V. alpha 2-Macroglobulin, complement, and biologic defense: antigens, growth factors, microbial proteases, and receptor ligation. Lab Invest, 71: 792-812, 1994.
10. Sinnreich, O., Kratzsch, J., Reichenbach, A., Glaser, C., Huse, K., and Birkenmeier, G. Plasma levels of transforming growth factor-1beta and alpha2-macroglobulin before and after radical prostatectomy: association to clinicopathological parameters. Prostate, 61: 201-208, 2004.
11. Williams, S. E., Ashcom, J. D., Argraves, W. S., and Strickland, D. K. A novel mechanism for controlling the activity of alpha 2-macroglobulin receptor/low density lipoprotein receptor-related protein. Multiple regulatory sites for 39-kDa receptor-associated protein. J Biol Chem, 267: 9035-9040, 1992.
12. Pinner, S. and Sahai, E. Imaging amoeboid cancer cell motility in vivo. J Microsc, 231: 441-445, 2008.
13. Kunz-Schughart, L. A., Freyer, J. P., Hofstaedter, F., and Ebner, R. The use of 3-D cultures for high-throughput screening: the multicellular spheroid model. J Biomol Screen, 9: 273-285, 2004.

## Claims

1. A pharmaceutical composition comprising an LRP1 receptor agonist, wherein the LRP1 receptor agonist is selected from the group consisting of native or activated alpha-2-macroglobulin, a functional derivative or a functional fragment thereof and an antibody capable of binding to LRP1 receptor for use in the treatment of cancer selected from the group consisting of astrocytoma, glioblastoma, breast cancer, lung cancer, liver cancer, colon cancer, colorectal cancer, leukemia, sarcoma, mesotheleoma, prostate cancer, pancreatic cancer, cervical cancer, ovarian cancer, gastric cancer, esophageal cancer, head and neck cancer, melanoma, thyroid cancer and bone cancer, wherein said derivative is a protein with at least 80% sequence identity to said native or activated alpha-2-macroglobulin and/or is modified at the side chain or a free amino- or carboxy-terminus of an amino acid.

2. A pharmaceutical composition comprising an LRP1 receptor agonist, wherein the LRP1 receptor agonist is selected from the group consisting of native or activated alpha-2-macroglobulin, a functional derivative or a functional fragment thereof and an antibody capable of binding to LRP1 receptor for use in the treatment of an HIV infection, wherein said derivative is a protein with at least 80% sequence identity to said native or activated alpha-2-macroglobulin and/or is modified at the side chain or a free amino- or carboxy-terminus of an amino acid.

3. The pharmaceutical composition for use according to claim 1 or 2, comprising a further active compound suitable for the treatment of cancer and/or a further active compound for the treatment of an HIV infection.

4. Use of an LRP1 receptor agonist selected from the group consisting of native or activated alpha-2-macroglobulin, a functional derivative or a functional fragment thereof and an antibody capable of binding to LRP1 receptor, wherein said derivative is a protein with at least 80% sequence identity to said native or activated alpha-2-macroglobulin and/or is modified at the side chain or a free amino- or carboxy-terminus of an amino acid, or use of a pharmaceutical composition according to any of claims 1 to 3 for the manufacture of a medicament for the treatment of:
(i) cancer, wherein the cancer is selected from the group consisting of astrocytoma, glioblastoma, breast cancer, lung cancer, colon cancer, colorectal cancer, leukemia, sarcoma, mesotheleoma, prostate cancer, pancreatic cancer, cervical cancer, ovarian cancer, gastric cancer, esophageal cancer, head and neck cancer, melanoma, thyroid cancer and bone cancer; and/or
(ii) an HIV infection.

5. Use according to claim 4 and/or the pharmaceutical composition for use according to claims 1 to 3, wherein the antibody binds to the same or an overlapping binding site on the LRP1 receptor as activated alpha-2-macroglobulin.

6. An in vitro method for identifying a therapeutic agent for treating cancer, wherein the cancer is selected from the group consisting of astrocytoma, glioblastoma, breast cancer, lung cancer, liver cancer, colon cancer, colorectal cancer, leukemia, sarcoma, mesotheleoma, prostate cancer, pancreatic cancer, cervical cancer, ovarian cancer, gastric cancer, esophageal cancer, head and neck cancer, melanoma, thyroid cancer and bone cancer, the method comprising the steps of:
(a) providing at least one compound to be tested for its suitability as a therapeutic agent for treating said cancer;
(b) providing at least one cancer cell, wherein the cancer is selected from the group consisting of astrocytoma, glioblastoma, breast cancer, lung cancer, liver cancer, colon cancer, colorectal cancer, leukemia, sarcoma, mesotheleoma, prostate cancer, pancreatic cancer, cervical cancer, ovarian cancer, gastric cancer, esophageal cancer, head and neck cancer, melanoma, thyroid cancer and bone cancer;
(c) contacting said at least one cancer cell with said compound; and
(d) selecting the compound as the therapeutic agent if the compound binds to the LRP1 receptor expressed by the at least one cancer cell and if the binding of the compound to the LRP1 receptor results in downregulation or inhibition of the Wnt signaling pathway in the at least one cancer cell.

7. The method according to claim 6, wherein the compound to be tested is an antibody.

8. The method according to claim 6, wherein the cancer cell is an astrocytoma cell or a hematopoetic cancer cell.

9. A kit of parts comprising the pharmaceutical composition for use according to any of claims 1 to 3 or 5 and diagnostic reagents suitable for determining whether the Wnt-signaling pathway is up-regulated or constitutively active in a cell.

10. A kit of parts according to claim 9, wherein the diagnostic reagents suitable for determining whether the Wnt-signaling pathway is up-regulated or constitutively active in a cell are selected from the group consisting of a Wnt reporter plasmid, a luciferase reporter plasmid and an oligonucleotide primer pair suitable for quantifying the expression of a Wnt signaling pathway target gene by real-time PCR.

## Patentansprüche

1. Eine pharmazeutische Zusammensetzung umfassend einen LRP1 Rezeptoragonisten, wobei der LRP1 Rezeptoragonist ausgewählt ist aus der Gruppe bestehend aus nativem oder aktiviertem Alpha-2-Makroglobulin, einem funktionellen Derivat oder einem funktionellen Fragment davon und einem Antikörper, der in der Lage ist, an einen LRP1 Rezeptor zu binden, zur Verwendung bei der Behandlung von Krebs ausgewählt aus der Gruppe bestehend aus einem Astrozytom, Glioblastom, Brustkrebs, Lungenkrebs, Leberkrebs, Darmkrebs, Dickdarmkrebs, Leukämie, Sarkom, Mesotheleom, Prostatakrebs, Bauchspeicheldrüsenkrebs, Gebärmutterhalskrebs, Eierstrockkrebs, Magenkrebs, Speiseröhrenkrebs, Kopf- und Halskrebs, Melanom, Schilddrüsenkrebs und Knochenkrebs, wobei besagtes Derivat ein Protein mit mindestens 80% Sequenzidentität zu besagtem nativen oder aktivierten Alpha-2-Makroglobulin ist und/oder an der Seitenkette oder einem freien Amino- oder Carboxy-Ende einer Aminosäure modifiziert ist.

2. Eine pharmazeutische Zusammensetzung umfassend einen LRP1 Rezeptoragonisten, wobei der LRP1 Rezeptoragonist ausgewählt ist aus der Gruppe bestehend aus nativem oder aktiviertem Alpha-2-Makroglobulin, einem funktionellen Derivat oder einem funktionellen Fragment davon und einem Antikörper, der in der Lage ist, an einen LRP1 Rezeptor zu binden, zur Verwendung bei der Behandlung einer HIV Infektion, wobei besagtes Derivat ein Protein mit mindestens 80% Sequenzidentität zu besagtem nativen oder aktivierten Alpha-2-Makroglobulin ist und/oder an der Seitenkette oder einem freien Amino- oder Carboxy-Terminus einer Aminosäure modifiziert ist.

3. Die pharmazeutische Zusammensetzung zur Verwendung gemäß Anspruch 1 oder 2, umfassend eine weitere aktive Verbindung, die sich zur Krebsbehandlung eignet, und/oder eine weitere aktive Verbindung zur Behandlung einer HIV Infektion.

4. Verwendung eines LRP1 Rezeptoragonisten ausgewählt aus der Gruppe bestehend aus nativem oder aktiviertem Alpha-2-Makroglobulin, einem funktionellen Derivat oder einem funktionellen Fragment davon und einem Antikörper, der in der Lage ist, an einen LRP1 Rezeptor zu binden, wobei besagtes Derivat ein Protein mit mindestens 80% Sequenzidentität zu besagtem nativen oder aktivierten Alpha-2-Makroglobulin ist und/oder an der Seitenkette oder einem freien Amino- oder Carboxy-Ende einer Aminosäure modifiziert ist, oder eine pharmazeutische Zusammensetzung gemäß einem der Ansprüche 1 bis 3, zur Herstellung eines Medikaments zur Behandlung von:
(i) Krebs, wobei der Krebs ausgewählt ist aus der Gruppe bestehend aus einem Astrozytom, Glioblastom, Brustkrebs, Lungenkrebs, Darmkrebs, Dickdarmkrebs, Leukämie, Sarkom, Mesotheleom, Prostatakrebs, Bauchspeicheldrüsenkrebs, Gebärmutterhalskrebs, Eierstockkrebs, Magenkrebs, Speiseröhrenkrebs, Kopf- und Halskrebs, Melanom, Schilddrüsenkrebs und Knochenkrebs; und/oder
(ii) einer HIV Infektion.

5. Verwendung gemäß Anspruch 4 und/oder die pharmazeutische Zusammensetzung zur Behandlung gemäß einem der Ansprüche 1 bis 3, wobei der Antikörper an dieselbe oder eine überlappende Bindungsstelle an den LRP1 Rezeptor wie aktiviertes Alpha-2-Makroglobulin bindet.

6. Ein in vitro Verfahren zur Identifikation eines therapeutischen Wirkstoffes zur Behandlung von Krebs, wobei der Krebs ausgewählt ist aus der Gruppe bestehend aus einem Astrozytom, Glioblastom, Brustkrebs, Lungenkrebs, Leberkrebs, Darmkrebs, Dickdarmkrebs, Leukämie, Sarkom, Mesotheleom, Prostatakrebs, Bauchspeicheldrüsenkrebs, Gebärmutterhalskrebs, Eierstockkrebs, Magenkrebs, Speiseröhrenkrebs, Kopf- und Halskrebs, Melanom, Schilddrüsenkrebs und Knochenkrebs, das Verfahren umfassend die Schritte:
(a) Bereitstellung mindestens einer Verbindung, die hinsichtlich ihrer Eignung als ein therapeutischer Wirkstoff zur Behandlung besagten Krebses geprüft wird;
(b) Bereitstellung mindestens einer Krebszelle, wobei der Krebs ausgewählt ist aus der Gruppe bestehend aus einem Astrozytom, Glioblastom, Brustkrebs, Lungenkrebs, Leberkrebs, Darmkrebs, Dickdarmkrebs, Leukämie, Sarkom, Mesotheleom, Prostatakrebs, Bauchspeicheldrüsenkrebs, Gebärmutterhalskrebs, Eierstockkrebs, Magenkrebs, Speiseröhrenkrebs, Kopf- und Halskrebs, Melanom, Schilddrüsenkrebs und Knochenkrebs;
(c) in Kontakt bringen besagter mindestens einer Krebszelle mit besagter Verbindung; und
(d) Auswahl der Verbindung als des therapeutischen Wirkstoffs wenn die Verbindung an den von der mindestens einen Krebszelle exprimierten LRP1 Rezeptor bindet und wenn die Bindung der Verbindung an den LRP1 Rezeptor in der Herunterregulation oder Hemmung des Wnt Signalweges in der mindestens einen Krebszelle führt.

7. Das Verfahren gemäß Anspruch 6, wobei die zu prüfende Verbindung ein Antikörper ist.

8. Das Verfahren gemäß Anspruch 6, wobei die Krebszelle eine Astrozytomzelle oder eine hematopeotische Krebszelle ist.

9. Ein Kit umfassend die pharmazeutische Zusammensetzung zur Verwendung gemäß einem der Ansprüche 1 bis 3 oder 5 und diagnostische Reagenzien, die sich zur Feststellung eignen, ob der Wnt-Signalweg in einer Zelle hoch reguliert oder konstitutiv aktiv ist.

10. Ein Kit gemäß Anspruch 9, wobei die diagnostischen Reagenzien, die sich zur Bestimmung eignen, ob der Wnt-Signalweg in einer Zelle hoch reguliert oder konstitutiv aktiv ist, ausgewählt sind aus der Gruppe bestehend aus einem Wnt-Reporterplasmid, einem Luziferase-Reporterplasmid und einem Oligonukleotid Primerpaar, das sich für die Quantifizierung der Expression eines Zielgens des Wnt Signalwegs durch Real-Time PCR eignet.

## Revendications

1. Composition pharmaceutique comprenant un agoniste du récepteur LRP1, dans laquelle l'agoniste du récepteur LRP1 est choisi dans le groupe constitué par l'alpha-2-macroglobuline native ou activée, un dérivé fonctionnel ou un fragment fonctionnel de celle-ci et un anticorps en mesure de se fixer au récepteur LRP1 pour une utilisation dans le traitement de cancers choisis dans le groupe constitué par l'astrocytome, le glioblastome, le cancer du sein, le cancer du poumon, le cancer du foie, le cancer du côlon, le cancer colorectal, la leucémie, le sarcome, le mésothéliome, le cancer de la prostate, le cancer du pancréas, le cancer du col de l'utérus, le cancer des ovaires, le cancer de l'estomac, le cancer de l'oesophage, le cancer des voies aérodigestives supérieures, le mélanome, le cancer de la thyroïde et le cancer des os, dans laquelle ledit dérivé est une protéine avec une séquence identique au moins à 80 % à ladite alpha-2-macroglobuline native ou activée et/ou est modifiée au niveau de la chaîne latérale ou au niveau d'une terminaison amine ou carboxy libre d'un acide aminé.

2. Composition pharmaceutique comprenant un agoniste du récepteur LRP1, dans laquelle l'agoniste du récepteur LRP1 est choisi dans le groupe constitué par l'alpha-2-macroglobuline native ou activée, un dérivé fonctionnel ou un fragment fonctionnel de celle-ci et un anticorps en mesure de se fixer au récepteur LRP1 pour une utilisation dans le traitement d'une infection par le VIH, dans laquelle ledit dérivé est une protéine avec une séquence identique au moins à 80 % à ladite alpha-2-macroglobuline native ou activée et/ou est modifiée au niveau de la chaîne latérale ou au niveau d'une terminaison amine ou carboxy libre d'un acide aminé.

3. Composition pharmaceutique pour une utilisation selon la revendication 1 ou 2, comprenant un autre composé actif adapté pour le traitement du cancer et/ou un autre composé actif pour le traitement d'une infection par le VIH.

4. Utilisation d'un agoniste du récepteur LRP1 choisi dans le groupe constitué par l'alpha-2-macroglobuline native ou activée, un dérivé fonctionnel ou un fragment fonctionnel de celle-ci et un anticorps en mesure de se fixer au récepteur LRP1, dans laquelle ledit dérivé est une protéine avec une séquence identique au moins à 80 % à ladite alpha-2-macroglobuline native ou activée et/ou est modifiée au niveau de la chaîne latérale ou au niveau d'une terminaison amine ou carboxy libre d'un acide aminé, ou l'utilisation d'une composition pharmaceutique selon l'une quelconque des revendications 1 à 3 pour la fabrication d'un médicament destiné au traitement du :
(i) cancer, le cancer étant choisi dans le groupe constitué par l'astrocytome, le glioblastome, le cancer du sein, le cancer du poumon, le cancer du foie, le cancer du côlon, le cancer colorectal, la leucémie, le sarcome, le mésothéliome, le cancer de la prostate, le cancer du pancréas, le cancer du col de l'utérus, le cancer des ovaires, le cancer de l'estomac, le cancer de l'oesophage, le cancer des voies aérodigestives supérieures, le mélanome, le cancer de la thyroïde et le cancer des os ; et/ou
(ii) une infection par le VIH.

5. Utilisation selon la revendication 4, et/ou composition pharmaceutique pour une utilisation selon les revendications 1 à 3, dans laquelle l'anticorps se fixe au même site de liaison ou à un site de liaison par recouvrement sur le récepteur LRP1 en tant qu'alpha-2-macroglobuline activée.

6. Procédé in vitro pour identifier un agent thérapeutique destiné au traitement du cancer, dans lequel le cancer est choisi dans le groupe constitué par l'astrocytome, le glioblastome, le cancer du sein, le cancer du poumon, le cancer du foie, le cancer du côlon, le cancer colorectal, la leucémie, le sarcome, le mésothéliome, le cancer de la prostate, le cancer du pancréas, le cancer du col de l'utérus, le cancer des ovaires, le cancer de l'estomac, le cancer de l'oesophage, le cancer des voies aérodigestives supérieures, le mélanome, le cancer de la thyroïde et le cancer des os, le procédé comprenant les étapes de :
(a) mise à disposition d'au moins un composé à tester pour sa pertinence en tant qu'agent thérapeutique dans le traitement dudit cancer ;
(b) mise à disposition d'au moins une cellule cancéreuse, le cancer étant choisi dans le groupe constitué par l'astrocytome, le glioblastome, le cancer du sein, le cancer du poumon, le cancer du foie, le cancer du côlon, le cancer colorectal, la leucémie, le sarcome, le mésothéliome, le cancer de la prostate, le cancer du pancréas, le cancer du col de l'utérus, le cancer des ovaires, le cancer de l'estomac, le cancer de l'oesophage, le cancer des voies aérodigestives supérieures, le mélanome, le cancer de la thyroïde et le cancer des os ;
(c) mise en contact de ladite au moins une cellule cancéreuse avec ledit composé ; et
(d) choix du composé en tant qu'agent thérapeutique si le composé se fixe au récepteur LRP1 exprimé par l'au moins une cellule cancéreuse et si la fixation du composé au récepteur LRP1 est le résultat d'une diminution ou de l'inhibition de la voie de signalisation Wnt dans l'au moins une cellule cancéreuse.

7. Procédé selon la revendication 6, dans lequel le composé à tester est un anticorps.

8. Procédé selon la revendication 6, dans lequel la cellule cancéreuse est une cellule d'astrocytome ou une cellule cancéreuse hématopoïétique.

9. Trousse de produits comprenant la composition pharmaceutique pour une utilisation selon l'une quelconque des revendications 1 à 3 ou 5 et les réactifs de diagnostic adaptés pour déterminer si la voie de signalisation Wnt est en augmentation ou est constitutivement active dans une cellule.

10. Trousse de produits selon la revendication 9, dans laquelle les réactifs de diagnostic adaptés pour déterminer si la voie de signalisation Wnt est en augmentation ou est constitutivement active dans une cellule sont choisis dans le groupe constitué par un plasmide rapporteur de Wnt, un plasmide rapporteur de luciférase et une paire d'amorces oligonucléotidiques adaptés pour quantifier l'expression d'un gène cible de la voie de signalisation Wnt par PCR (réactions en chaîne de la polymérase) en temps réel.
